# EUROPÄISCHE PATENTSCHRIFT

(11) **EP 1 608 278 B1**
(45) Veröffentlichungstag und Bekanntmachung des Hinweises auf die Patenterteilung: **02.12.2009**
(21) Anmeldenummer: 04723486.9
(22) Anmeldetag: 26.03.2004
(51) Int. Cl.: A61B 17/80

(54) **AUFNAHME FÜR EIN VERBLOCKUNGSELEMENT UND VERBLOCKUNGSELEMENT**
HOUSING FOR A LOCKING ELEMENT AND LOCKING ELEMENT
LOGEMENT POUR UN ELEMENT DE BLOCAGE ET ELEMENT DE BLOCAGE

(30) Priorität: 03.04.2003 CH 5922003; 23.09.2003 CH 16212003
(43) Veröffentlichungstag der Anmeldung: 28.12.2005
(73) Patentinhaber: Medartis AG, 4057 Basel (CH)
(72) Erfinder: PFEFFERLE, Joachim, 79244 Münstertal (DE); ZEUNER, Hermann, 79111 Freiburg (DE); SCHEUBLE, Peter, 79418 Schliengen (DE); WALTER, Matthias, 79312 Emmendingen (DE); THIEL, Dirk, 79219 Staufen (DE)
(74) Vertreter: Müller, Christoph Emanuel
(86) Internationale Anmeldenummer: PCT/CH2004/000188
(87) Internationale Veröffentlichungsnummer: WO 2004/086990

(56) Entgegenhaltungen:
- EP-A- 1 093 762
- WO-A-00/66012
- WO-A-94/07040

## Beschreibung

Die Erfindung betrifft eine Aufnahme für ein Verblockungs-element sowie ein Verblockungselement gemäss dem Oberbegriff des jeweiligen unabhängigen Patentanspruchs.

Solche Aufnahmen und Verblockungselemente haben von ihrem Typ her im weitesten Sinne eine Anwendung bei der Verbindung von verschiedenen Elementen, wo immer solche Elemente miteinander fest verbunden werden sollen, z.B. im allgemeinen Maschinenbau (so z.B. bei Welle-Nabe-Verbindungen, wie sie aus der DE-A-42 09 153 bekannt sind, oder bei sonstigen Verbindungen, z.B. bei Schraubverbindungen, wo zwei Bauteile miteinander fest verblockt werden sollen). Das Dokument WO 94/07040 offenbart eine Vorrichtung zum Verbinden von zwei Elementen. Der Oberbegriff von Anspruch 1 basiert auf diesem Dokument. Ein spezielles Anwendungsgebiet solcher Verbindungen ist das Gebiet der Medizintechnik, und hier beispielsweise das Gebiet der Traumatologie (Unfallmedizin), wo es nicht selten um die Fixierung von Knochensegmenten geht, beispielsweise bei Frakturen. Dabei müssen für eine optimale Versorgung der Frakturen die einzelnen Knochensegmente möglichst genau repositioniert und mit Hilfe einer Knochenplatte und Knochenschrauben in der gewünschten Position fixiert werden, damit der Behandlungserfolg maximal ist.

Für den Behandlungserfolg ist es wichtig, dass die Knochensegmente nach der Fixierung mit Hilfe der Knochenplatte stabil angeordnet bleiben, es darf sich also nicht etwa eine Knochenschraube aus der Knochenplatte lösen, was z.B. bei einer ungünstigen Belastung einer versorgten Fraktur in seltenen Fällen vorkommen kann. In solchen Fällen können dann die Knochensegmente eine von der gewünschten Position verschiedene Position einnehmen, was den Behandlungserfolg verringern kann.

In der WO-A-00/66012 ist bereits eine Knochenplatte vorgeschlagen worden, bei der im Plattenloch eine Eingriffkontur vorgesehen ist, welche mehrere horizontal und radial verlaufende Konturtäler und dazu benachbarte Konturspitzen aufweist. Die Eingriffskontur läuft jedoch nicht vollständig im Plattenloch um, sondern läuft jeweils an zwei horizontal gegenüberliegenden Enden aus. Dort verbleiben im Plattenloch unkonturierte Wandbereiche, wodurch das Plattenloch langlochförmig ist. Die zugehörige Knochenschraube weist an ihrem Schaft ein Knochengewinde auf, und oberhalb des Knochengewindes, aber unterhalb des Schraubenkopfs, ein zusätzliches Verblockungsgewinde. Während das Knochengewinde in gewohnter Weise in das Knochensegment eingeschraubt wird, greift in der Schlussphase des Eindrehens der Knochenschraube das Verblockungsgewinde in die Eingriffskontur des Plattenlochs ein. Da das Verblockungsgewinde mit seinem wendelförmigen Verlauf und seiner Steigung nicht komplementär zu der Eingriffskontur im Plattenloch ist, kommt es zu einer Deformation an beiden und somit zu einer widerstandsfähigen Verbindung von Knochenplatte und Knochenschraube, welche zwar grundsätzlich wieder lösbar ist, jedoch ein selbsttätiges Lösen der Schraube zuverlässig verhindert.

Werden also beispielsweise zwei Knochensegmente, ein erstes Knochensegment diesseits einer Fraktur und ein zweites Knochensegment jenseits der Fraktur relativ zueinander in eine gewünschte Position gebracht, wird dann in jedes der beiden Knochensegmente durch das Plattenloch der Knochenplatte hindurch eine Knochenschraube eingeschraubt, und die Knochenschrauben im Plattenloch der Knochenplatte verblockt, so bleiben nach dem Verblocken die Knochensegmente in der gewünschten Position. Die stabile Verblockung im Plattenloch erlaubt dabei unter anderem auch, dass die Knochenplatte nicht auf dem Knochen aufliegen muss, sondern z.B. auch in geringem Abstand vom Knochen stabil angeordnet sein kann. Dies kann von Vorteil sein, weil die Knochenhaut für die bessere Durchblutung/Ernährung des Knochens sorgt und somit eine intakte Knochenhaut den Heilungsprozess fördert. Diese in der WO-A-00/66012 beschriebene sehr funktionstüchtige Knochenplatte erlaubt das Eindrehen der Schraube mit einer gewissen Abweichung von der senkrechten Einschraubachse, die senkrechte Achse des Plattenlochs und die tatsächliche Einschraubrichtung können also einen gewissen Winkel miteinander einschliessen. Allerdings kann eine solche Abweichung der tatsächlichen Einschraubachse von der senkrechten Achse des Plattenlochs (Verkippung der tatsächlichen Einschraubachse gegenüber der senkrechten Achse des Plattenlochs) nur in Richtung der längeren Achse des langlochförmigen Plattenlochs (also in Richtung auf die beiden Enden des Plattenlochs mit den unkonturierten Wandbereichen zu) erfolgen, und sie ist nur innerhalb eines relativ kleinen Winkelbereichs möglich.

Vielfach wäre es für den operierenden Arzt jedoch von Vorteil, wenn er eine Knochenschraube möglichst frei im Hinblick auf die Richtung setzen kann, z.B. wenn eine zu behandelnde Fraktur ausserhalb des Zugangswegs liegt, oder wenn das Angebot an Knochenmaterial so ist, dass die Schraube am besten in einer bestimmten Richtung gesetzt werden sollte.

Zu bedenken ist ferner, dass in manchen Fällen eine nachträgliche Neupositionierung eines Knochensegments erwünscht sein kann. Dies ist bei der aus der WO-A-00/66012 bekannten Knochenplatte zwar grundsätzlich möglich, eine solche Neupositionierung ist jedoch wegen des Umformens von Verblockungsgewinde und Eingriffskontur schwieriger. Gerade bei komplizierten Frakturen mit mehreren und unter Umständen kleinen Knochensegmenten - wie z.B. im Bereich der Hand - ist es aber häufig sehr wichtig im Sinne einer optimalen Positionierung der einzelnen Knochensegmente, dass die Verblockung ohne Beschädigung der Schraube und/oder der Platte wieder gelöst werden kann und einzelne Knochensegmente in gewissem Umfang anders positioniert werden können.

Hier setzt die vorliegende Erfindung an, deren generelle Aufgabe es ist, eine Verbindung - also sowohl eine Aufnahme als auch ein entsprechendes Verblockungselement - vorzuschlagen, die hochflexibel und gleichzeitig zuverlässig ist. Insbesondere soll eine Verblockung in verschiedenen Richtungen und unter verschiedenen Winkeln (also nicht nur in einer Richtung) möglich sein. Ausserdem soll es auch möglich sein, nach einer erstmaligen Verblockung die Verblockung einfach wieder zu lösen zu können und anschliessend wieder eine neue Verblockung vornehmen zu können, ohne dass dabei die Aufnahme (z.B. das Plattenloch der Knochenplatte) oder das Verblockungselement (z.B. die Schraube) beschädigt werden. Gleichzeitig soll beim Verblocken eine zuverlässige Verbindung hergestellt werden, die ein selbsttätiges Lösen des Verblockungselements (z.B. der Schraube) nicht zulässt.

Diese Aufgabe wird erfindungsgemäss durch eine Aufnahme und durch ein entsprechendes Verblockungselement gelöst, wie sie durch die Merkmale des jeweiligen unabhängigen Patentanspruchs charakterisiert sind, sowie durch eine Verbindung einer solchen Aufnahme mit einem solchen Verblockungselement. Zu erwähnen ist dabei, dass bei einer solchen Verbindung die Aufnahme zumindest im Bereich der sich erweiternden Ausnehmung in Richtung der Längsachse der Aufnahme betrachtet auch zylindrisch ausgebildet sein kann. Vorteilhafte Ausgestaltungen ergeben sich aus den Merkmalen der abhängigen Patentansprüche. Während das Spektrum der möglichen Anwendungen grundsätzlich sehr breit ist - wie eingangs bereits erwähnt - liegt eine Anwendung von besonderer Bedeutung in der Bereitstellung von Knochenplatten, bei denen in einem oder mehreren Plattenlöchern derartige Aufnahmen vorgesehen sind, sowie in der Bereitstellung entsprechender Knochenschrauben als Verblockungselemente, und schliesslich in der Bereitstellung entsprechender Sortimente von Knochenplatten/Knochenschrauben.

Insbesondere ist bei der erfindungsgemässen Aufnahme die umlaufende Innenwand in Richtung der Längsachse der Aufnahme betrachtet zumindest im Bereich der Ausnehmung annähernd sphärisch, parabolisch, elliptisch oder hyperbolisch ausgebildet. Dies erlaubt eine Verkippung der Längsachse des Verblockungsteils des Verblockungselements, dessen umlaufende Aussenfläche dementsprechend wenigstens im Bereich der zugehörigen Klemmfläche zumindest annähernd sphärisch ausgebildet ist, relativ zur Längsachse der Aufnahme. Die Längsachse des Verblockungsteils des Verblockungselements und die Längsachse der Aufnahme können also einen Winkel einschliessen. Trotzdem ist auch unter diesem Winkel eine zuverlässige Verblockung möglich, die ein selbsttätiges Lösen des Verblockungsteils und damit des Verblockungselements verhindert, die jedoch bei Bedarf auch wieder gelöst werden kann ohne Beschädigung der Aufnahme und/oder des Verblockungselements (bzw. dessen Verblockungsteil). Dabei lässt sich das Verblockungsteil stufenlos verkippen und kann mit der jeweiligen Verkippung wieder zuverlässig verblockt werden. Vorteilhaft ist auch, dass es sich bei der Aufnahme und dem Verblockungsteil jeweils nur um eine einzige Komponente handelt. So kann die Aufnahme direkt z.B. in einem Plattenloch einer Knochenplatte vorgesehen werden und der Verblockungsteil kann direkt am Schraubenkopf einer Knochenschraube ausgebildet sein, sodass ausser der ohnehin erforderlichen Knochenplatte und der ohnehin erforderlichen Knochenschraube keine weiteren Komponenten notwendig sind.

Bei einem vorteilhaften Ausführungsbeispiel der Aufnahme kann die Ausnehmung so angeordnet sein, dass sie in einer Richtung senkrecht zur Längsachse der Aufnahme (also z.B. horizontal) umläuft. Bei einem anderen vorteilhaften Ausführungsbeispiel kann die Ausnehmung so angeordnet sein, dass sie nach Art einer räumlichen Spirale um die Längsachse der Aufnahme umläuft.

Bei einem vorteilhaften Ausführungsbeispiel der Aufnahme kann die umlaufende Innenwand drei gleichmässig entlang ihres Umfangs verteilte Ausnehmungen mit einer sich keilförmig nach aussen von der Längsachse der Aufnahme weg erweiternden Ausnehmung aufweisen. Diese drei (jeweils um 120° versetzt angeordneten) Ausnehmungen bewirken eine gleichmässige Belastung der Aufnahme und auch des zugehörigen Verblockungsteils (Prinzip des "Dreibeins").

Besonders vorteilhaft kann eine Ausgestaltung sein, bei der die in Richtung der Längsachse zumindest annähernd sphärische Kontur der Innenwand im Bereich der keilförmig sich nach aussen von der Längsachse der Aufnahme weg erweiternden Ausnehmung bzw. Ausnehmungen durch einen Teil einer Funktion vom Typ r = a₁ + b₁√α (bei einem räumlich spiralförmigen Verlaufe kommt in Längsrichtung betrachtet noch eine Komponente c₁ x α zum Tragen) beschrieben wird, wobei r den jeweiligen Abstand der Innenwand von der Längsachse der Aufnahme bedeutet, a₁ und b₁Konstanten in der Ebene und c₁ eine Konstante in der Längsrichtung sind, und α für den jeweiligen Umlaufwinkel steht. Dieser Typ von Funktion beschreibt eine Kontur, bei der sich der jeweils aktuelle Abstand r eines Punkts auf der Innenwand von der Längsachse ergibt aus einem konstanten Abstand a₁ von der Längsachse zuzüglich eines Abstands b₁√α, also zuzüglich eines Abstandes, der sich einer Wurzelfunktion folgend in Abhängigkeit vom Umlaufwinkel vergrössert (sonst würde sich dieser Bereich nicht von der Längsachse weg erweitern). Bei einem räumlichen Verlauf ist diesem Verlauf noch eine Komponente in Längsrichtung überlagert. Bei einer solchen Kontur und bei entsprechend geeigneten Parametern a₁ und b₁ (bzw. c₁) kommt einerseits eine zuverlässige Verblockung zustande, andererseits lässt sich die Verbindung auch ohne Beschädigung wieder lösen.

Alternativ kann sich die in Richtung der Längsachse betrachtet zumindest annähernd sphärische Kontur der Innenwand im Bereich der keilförmig sich nach aussen von der Längsachse der Aufnahme weg erweiternden Ausnehmung bzw. Ausnehmungen durch einen Teil einer logarithmischen Spirale, durch einen Teil einer Kreisbahn, durch einen Teil einer Evolvente, oder ähnlich, beschrieben werden. Auch eine derartige Kontur erlaubt eine zuverlässige Verblockung einerseits, andererseits lässt sich die Verblockung auch ohne Beschädigung wieder lösen.

Bei einem weiteren Ausführungsbeispiel der Aufnahme kann diese an ihrer umlaufenden Innenwand eine in Umlaufrichtung an die Ausnehmung bzw. Ausnehmungen anschliessende Auslaufkontur aufweisen zum Ausleiten des Verblockungselements aus der Aufnahme. Diese Auslaufkontur dient der bequemeren Entnahme des Verblockungselements (z.B. der Schraube) aus der Aufnahme (z.B. dem Plattenloch). Besonders bei kleinen Teilen (z.B. kleinen Schrauben) kann dies das Handling sehr erleichtern.

Bei einem weiteren Ausführungsbeispiel der Aufnahme ist diese mit einer Ansenkung versehen zur Aufnahme eines Schraubenkopfs mit sphärischer Kopfunterseite. Dies ermöglicht beispielsweise auf dem bereits angesprochenen Anwendungsgebiet der Osteosynthese auch gewöhnliche Knochenschrauben mit sphärischer Kopfunterseite, aber ohne Verblockungskontur, einsetzen zu können. Der Kopf einer solchen Schraube wird dann von der Ansenkung in herkömmlicher Weise aufgenommen.

Wie bereits erwähnt, ist die Osteosynthese ein Anwendungsgebiet von besonderer Bedeutung. Daher ist Gegenstand der Erfindung auch eine Knochenplatte mit Plattenlöchern, wobei mindestens ein Plattenloch mit einer Aufnahme versehen ist für ein Verblockungselement, welches mit der Knochenplatte zu verblocken ist, z.B. für eine Knochenschraube, für einen Fixierstift, oder für eine Bohrführung. Die Aufnahme im Plattenloch ist dabei wie vorstehend beschrieben ausgebildet.

Dabei kann beispielsweise mindestens ein Plattenloch als Langloch ausgebildet sein, in welchem eine solche - wie vorstehend beschriebene - Aufnahme vorgesehen ist. Dies ermöglicht den Einsatz der Knochenplatte bei der Kompressionsosteosynthese, und bietet gleichzeitig die Vorteile der erfindungsgemässen Verblockung.

Bei dem erfindungsgemässen Verblockungselement ist die umlaufende Aussenfläche in Richtung der Längsachse betrachtet wenigstens im Bereich der Klemmfläche zumindest annähernd sphärisch, parabolisch, elliptisch oder hyperbolisch ausgebildet. Dies erlaubt eine Verkippung der Längsachse des Verblockungsteils des Verblockungselements relativ zur Längsachse der Aufnahme (die beiden Achsen schliessen einen Winkel ein). Eine Verblockung des Verblockungselements ist sogar dann möglich, wenn die Aufnahme im Bereich der Ausnehmung zylindrisch ausgebildet ist anstelle von zumindest annähernd sphärisch, parabolisch, elliptisch oder hyperbolisch. Es ist also auch unter einem Winkel eine zuverlässige Verblockung möglich, die ein selbsttätiges Lösen des Verblockungsteils und damit des Verblockungselements verhindert, die jedoch bei Bedarf auch wieder gelöst werden kann ohne Beschädigung der Aufnahme und/oder des Verblockungselements (bzw. dessen Verblockungsteil). Auch bei dem Verblockungselement kann die umlaufende Aussenfläche drei gleichmässig entlang ihres Umfangs verteilte Klemmflächen mit einer sich keilförmig nach aussen von der Längsachse weg erweiternden Klemmfläche aufweisen. Diese drei (jeweils um 120° versetzt angeordneten) Klemmflächen bewirken eine gleichmässige Belastung des Verblockungsteils des Verblockungselements und auch der Aufnahme des zugehörigen Verblockungsteils (Prinzip des "Dreibeins", siehe oben).

Bei dem erfindungsgemässen Verblockungselement kann die Kontur der Aussenfläche im Bereich der keilförmig sich nach aussen von der Längsachse weg erweiternden Klemmfläche bzw. Klemmflächen in einer Azimutebene durch eine Funktion vom Typ r = a₂ + b₂√α beschrieben werden, wobei r den jeweiligen Abstand der Klemmfläche von der Längsachse bedeutet, a₂ und b₂ Konstanten sind, und α für den jeweiligen Azimutwinkel steht. Die Vorteile einer solchen, einer Wurzelfunktion folgenden Kontur, sind bereits oben diskutiert worden.

Alternativ kann auch bei dem Verblockungselement die Kontur der Aussenfläche im Bereich der keilförmig sich nach aussen von der Längsachse weg erweiternden Klemmfläche bzw. Klemmflächen in einer Azimutebene durch einen Teil einer logarithmischen Spirale, durch einen Teil einer Kreisbahn, durch einen Teil einer Evolvente, oder ähnlich, beschrieben werden. Auch die Vorteile einer solchen Kontur sind bereits weiter oben schon beschrieben worden.

Zumindest die Klemmfläche (aber auch die komplette Aussenfläche des Verblockungsteils des Verblockungselements) kann bei einem Ausführungsbeispiel mit einer Struktur versehen sein. Eine solche (Fein-)Struktur kann die Verblockung noch verbessern.

Wie bereits ebenfalls erwähnt, ist ein Anwendungsfall von besonderer Bedeutung derjenige, bei welchem das Verblockungselement eine Schraube, insbesondere Knochenschraube, ist. Diese ist mit einem Schraubenschaft versehen, der seinerseits wenigstens teilweise mit einem Gewinde versehen ist, sowie mit einem über den Schaft und das Gewinde nach aussen vorstehenden Schraubenkopf. Der Schraubenkopf entspricht dabei dem Verblockungsteil des Verblockungselements, wie es vorstehend beschrieben ist.

Bei dieser Schraube können in den Klemmflächen mindestens eine, vorzugsweise vier, rillenförmige Vertiefungen vorgesehen sein, welche im wesentlichen in gleicher Richtung verlaufen und im wesentlichen die gleiche Steigung aufweisen wie das vom Schraubenschaft nach aussen vorstehende Gewinde. Durch das Vorsehen von derartigen Vertiefungen können unerwünschte Verklemmungen des Schraubenkopfs verhindert werden, die in sehr ungünstigen Fällen durch Verklemmen der Klemmfläche mit einem nach innen vorspringenden Bereich der Kontur der Aufnahme auftreten könnten. Ein derartiger nach innen vorspringender Bereich kann in einem solchen Fall in eine rillenförmigen Vertiefung hinein gleiten und wird beim weiteren Einschrauben der Schraube - durch den Verlauf und die Steigung der rillenförmigen Vertiefungen bedingt - von den rillenförmigen Vertiefungen einmalig durchfurcht. Nach dieser Durchfurchung läuft die Schraube wieder frei, d.h. es besteht kurzzeitig kein Kontakt zwischen Aufnahme und Schraubenkopf. Beim weiteren Einschrauben tritt dann die zuvor bereits beschriebene Verblockung ein. Trotz des Vorhandenseins der rillenförmigen Vertiefungen verbleibt eine genügend grosse Klemmfläche, um eine zuverlässige Verblockung des Schraubenkopfs in der Aufnahme der Knochenplatte zu gewährleisten.

Von besonderer Bedeutung ist schliesslich noch ein Knochenplatten-/Knochenschrauben- Sortiment, welches mindestens eine Knochenplatte wie vorstehend beschrieben sowie mindestens eine (vorzugsweise mehrere) Knochenschraube(n) wie vorstehend beschrieben umfasst. Diese Sortimente kommen regelmässig im Rahmen einer Versorgung von Frakturen zum Einsatz.

Wie weiter oben bereits erwähnt, betrifft ein wesentlicher Aspekt der Erfindung auch die Verbindung einer Aufnahme, wie sie vorstehend beschrieben ist, mit einem Verblockungselement, wie es vorstehend beschrieben ist.

Dabei kann die Aufnahme wenigstens im Bereich der Ausnehmung in Richtung der Längsachse der Aufnahme betrachtet sogar zylindrisch ausgebildet sein anstelle von annähernd sphärisch, parabolisch, elliptisch oder hyperbolisch. Gleichwohl kommt es zu einer zuverlässigen Verblockung, anderseits lässt sich die Verbindung bei Bedarf auch ohne Beschädigungen wieder lösen.

Auch eine derartige Verbindung hat ein besonderes Anwendungsgebiet in der Osteosynthese. Daher ist Gegenstand der Erfindung auch eine Verbindung einer Knochenplatte, wie sie vorstehend beschrieben ist, mit einer Schraube, wie sie vorstehend beschreiben ist.

Weitere vorteilhafte Ausgestaltungen der Erfindung ergeben sich aus der nachfolgenden Beschreibung von Ausführungsbeispielen der Erfindung mit Hilfe der schematischen Zeichnung. Es zeigen:
- Fig. 1: ein Ausführungsbeispiel eines erfindungsgemässen Verblockungselements in Form einer Knochenschraube in perspektivischer Ansicht,
- Fig. 2: die Knochenschraube aus Fig. 1 in einer Aufsicht,
- Fig. 3: ein Ausschnitt aus einer Knochenplatte mit einem Ausführungsbeispiel einer erfindungsgemässen Aufnahme im Plattenloch in einer perspektivischen Ansicht,
- Fig. 4: den Ausschnitt aus der Knochenplatte aus Fig. 3 in einer anderen perspektivischen Ansicht,
- Fig. 5: eine schematische Darstellung der Aussenkontur des Schraubenkopfes und der entsprechenden Innenkontur der Aufnahme, in unverblockter Position,
- Fig. 6: die Darstellung gemäss Fig. 5, jedoch in verblockter Position,
- Fig. 7: eine Schnittdarstellung einer im Plattenloch einer Knochenplatte angeordneten Schraube, wobei die Schraubenlängsachse mit der Längsachse des Plattenlochs zusammenfällt,
- Fig. 8: die Darstellung der Schraube im Plattenloch aus Fig. 7, wobei die Längsachse der Schraube und die Längsachse des Plattenlochs einen Winkel einschliessen,

- Fig. 9: eine Schnittdarstellung einer konventionellen Knochenschraube mit sphärischer Kopfunterseite im Plattenloch, wobei Schraubenlängsachse und Längsachse des Plattenlochs zusammenfallen,
- Fig. 10: die Schnittdarstellung aus Fig. 9, wobei jedoch die Schraubenlängsachse und die Längsachse des Plattenlochs einen Winkel einschliessen,
- Fig. 11: eine Knochenplatte, wie sie im Bereich der Handchirurgie zum Einsatz kommen kann, mit mehreren in den Plattenlöchern unter verschiedenen Winkeln darin angeordneten Schrauben,
- Fig.12-19: verschiedene Einzelschritte - Ausrichten, Fixieren, etc. - bei der Versorgung einer Fraktur,
- Fig. 20: ein Ausführungsbeispiel für einen Fräser, mit welchem die Innenkontur der Aufnahme hergestellt werden kann,
- Fig. 21: ein Ausführungsbeispiel für einen Fräser, mit welchem die zu Verblockung dienende Aussenkontur des Verblockungselements hergestellt werden kann,
- Fig. 22: die Knochenschraube aus Fig. 1, jedoch mit einer zusätzlichen Oberflächenstruktur auf der Aussenwand des Schraubenkopfs,
- Fig. 23: die Knochenschraube aus Fig. 22 im Längsschnitt,

- Fig. 24: die Knochenschraube aus Fig. 1, jedoch mit rillenförmigen Vertiefungen in der Klemmfläche des Schraubenkopfs,
- Fig. 25: ein weiteres Ausführungsbeispiel eines Verblockungselements in Form einer Bohrführung, welche z.B. im Plattenloch einer Knochenplatte verblockt werden kann,
- Fig. 26: ein weiteres Ausführungsbeispiel eines Verblockungselements in Form einer Knochenschraube, mit vier gleichmässig am Umfang des Schraubenkopfs angeordneten Klemmbereichen,
- Fig. 27: eine Aufsicht auf die Knochenschraube aus Fig. 26,
- Fig. 28: eine Aufsicht auf ein weiteres Ausführungsbeispiel einer Aufnahme in einem Langloch einer Knochenplatte, die z.B. bei der Kompressionsosteosynthese verwendet werden kann, mit zugehöriger Knochenschraube, in unverblockter Position, vor Beginn der Kompression,
- Fig. 29: das Ausführungsbeispiel aus Fig. 28 im Längsschnitt,
- Fig. 30: die Aufsicht entsprechend Fig. 28, wobei Schraube und Knochenplatte verblockt sind, in Kompressions-stellung,
- Fig. 31: den Längsschnitt entsprechend Fig. 29, wobei Schraube und Knochenplatte verblockt sind,
- Fig. 32: einen Längsschnitt durch die Aufnahme aus Fig. 31,
- und:
- Fig. 33: ein Ausschnitt aus einer Knochenplatte mit einem weiteren Ausführungsbeispiel einer erfindungsgemässen Aufnahme im Plattenloch in perspektivischer Ansicht (Verlauf der Ausnehmung nach Art einer räumlichen Spirale).

Die nachfolgenden Erläuterungen erfolgen für das spezielle Anwendungsgebiet von Knochenschrauben und Knochenplatten. Wie weiter oben bereits erwähnt, ist dies ein Anwendungsgebiet von besonderer Bedeutung, die Erfindung ist jedoch nicht auf dieses Anwendungsgebiet beschränkt, sondern betrifft ganz allgemein Verbindungen, die auf der nachfolgend anhand des Ausführungsbeispiels von Knochenschrauben und Knochenplatten detaillierter erläutert werden.

Ausserdem werden nachstehend nur solche Ausführungsbeispiele erläutert, bei denen sowohl die Aufnahme der Knochenplatte im Bereich der Ausnehmung als auch die Klemmfläche am Schraubenkopf im Bereich der Klemmfläche in Richtung der Längsachse betrachtet sphärisch ausgebildet sind. Ebenso kann die Ausnehmung aber beispielsweise auch annähernd parabolisch, elliptisch oder hyperbolisch ausgebildet sein. Dies gilt ebenfalls für die Klemmfläche. Die Ausnehmung kann sogar zylindrisch ausgebildet sein und mit einer annähernd sphärischen, parabolischen, elliptischen oder hyperbolischen Klemmfläche zusammen eine Verbindung eingehen, bei der es zu einer entsprechenden Verblockung kommt.

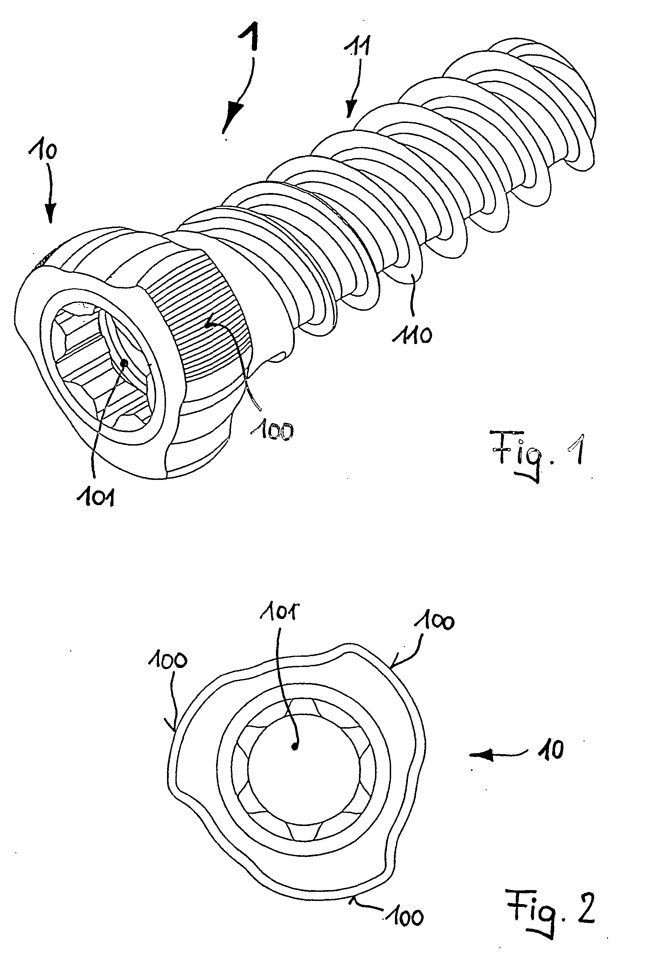

In **Fig. 1** erkennt man ein Ausführungsbeispiel eines erfindungsgemässen Verblockungselements in Form einer Knochenschraube 1. Der Verblockungsteil der Knochenschraube 1 wird hier durch den Schraubenkopf 10 gebildet. Dieser Schraubenkopf 10 ist anders (nämlich "unrund") ausgebildet als ein Schraubenkopf einer konventionellen Knochenschraube, er weist insbesondere Klemmflächen 100 auf - im hier gezeigten Ausführungsbeispiel sind dies drei gleichmässig entlang des Umfangs der Aussenfläche des Schraubenkopfs 10 angeordnete Klemmflächen 100. In einer Azimutebene betrachtet, also in einer Ebene senkrecht zur Längsachse der Schraube 1, erweitern sich diese Klemmflächen 100 keilförmig nach aussen hin, um eine Verblockung (Verklemmung) der Schraube 1 in einer entsprechenden Aufnahme einer Knochenplatte zu bewirken.

Ferner erkennt man in Fig. 1 im Schraubenkopf 10 eine Werkzeugaufnahme 101, die geeignet ist, ein Werkzeug (nicht dargestellt) aufzunehmen zum Eindrehen bzw. Ausdrehen der Schraube 1. Am Schraubenschaft 11 ist ein Schaftgewinde 110 vorgesehen, welches zum Eindrehen der Schraube 1 in den Knochen dient. Das Schaftgewinde 110 kann beispielsweise als selbstschneidendes Gewinde ausgebildet sein.

Die Aussenfläche des Schraubenkopfs ist wenigstens im Bereich der Klemmfläche 100, hier aber auch in den anderen Bereichen, in Längsrichtung der Schraube 1 betrachtet zumindest annähernd sphärisch ausgebildet. Auf diesen Aspekt wird später bei der Verblockung der Schraube 1 in einer Aufnahme noch einmal genauer eingegangen. In der Aufsicht in Fig. 2 erkennt man diese in Längsrichtung zumindest annähernd sphärische Kontur daran, dass ausser der Begrenzung der sich in Längsrichtung der Schraube 1 erstreckenden Aussenfläche am oberen Ende des Schraubenkopfs (innere Linie) auch noch der am weitesten nach aussen vorstehende Teil diese Aussenfläche (äussere Linie) zu erkennen ist.

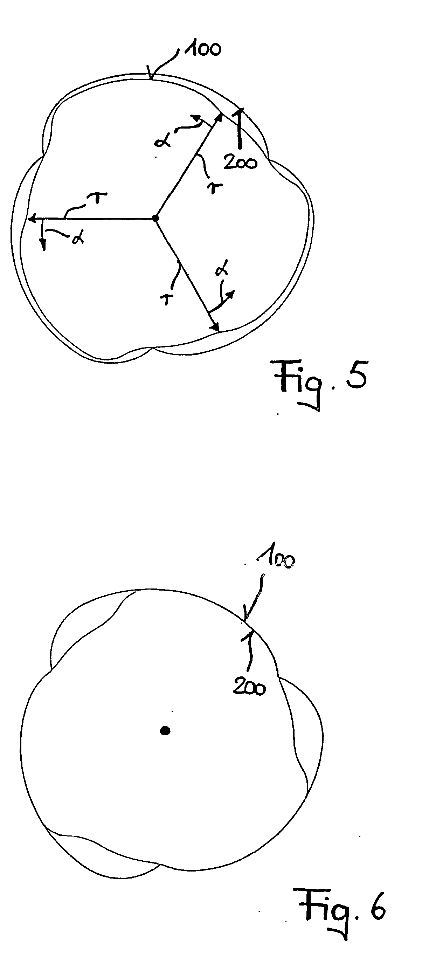

In einer Azimutebene - also in einer Ebene senkrecht zur Längsachse der Schraube 1 - kann die Kontur der sich keilförmig nach aussen erweiternden Klemmfläche 100 beispielsweise durch eine Funktion des Typs r = a₁ + b₁√α beschrieben werden (siehe hierzu auch Fig. 5), also durch eine "Wurzelfunktion". Dabei bedeutet r den Abstand der Klemmfläche von der Längsachse, a₁ und b₁ Konstanten, und α den jeweiligen Azimutwinkel. Die Klemmfläche 100 kann in der Azimutebene betrachtet auch durch einen Teil einer logarithmischen Spirale beschrieben werden. Auch andere beschreibende Funktionen kommen grundsätzlich in Betracht.

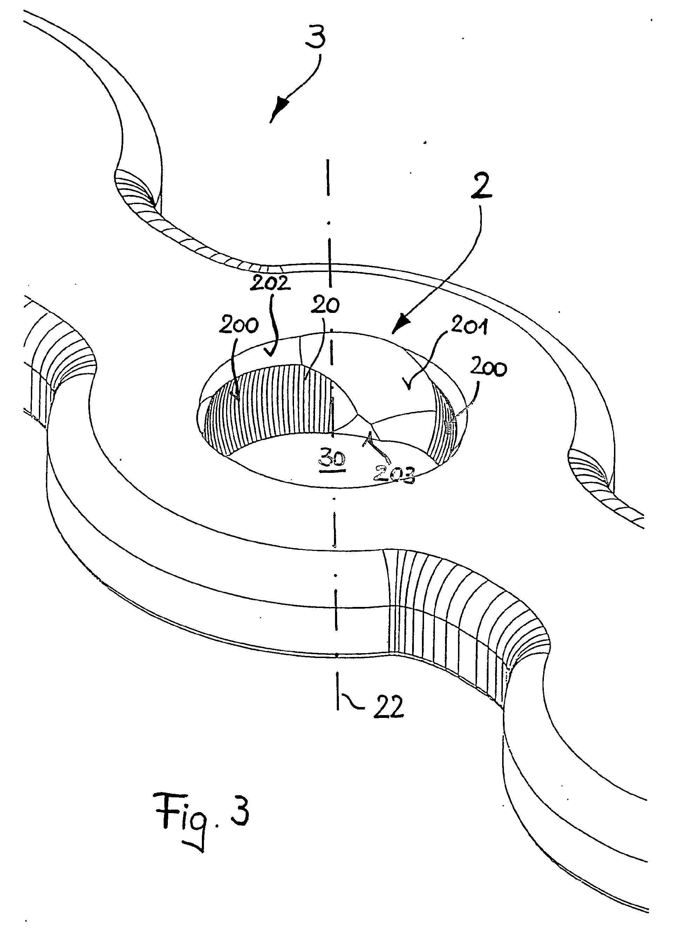

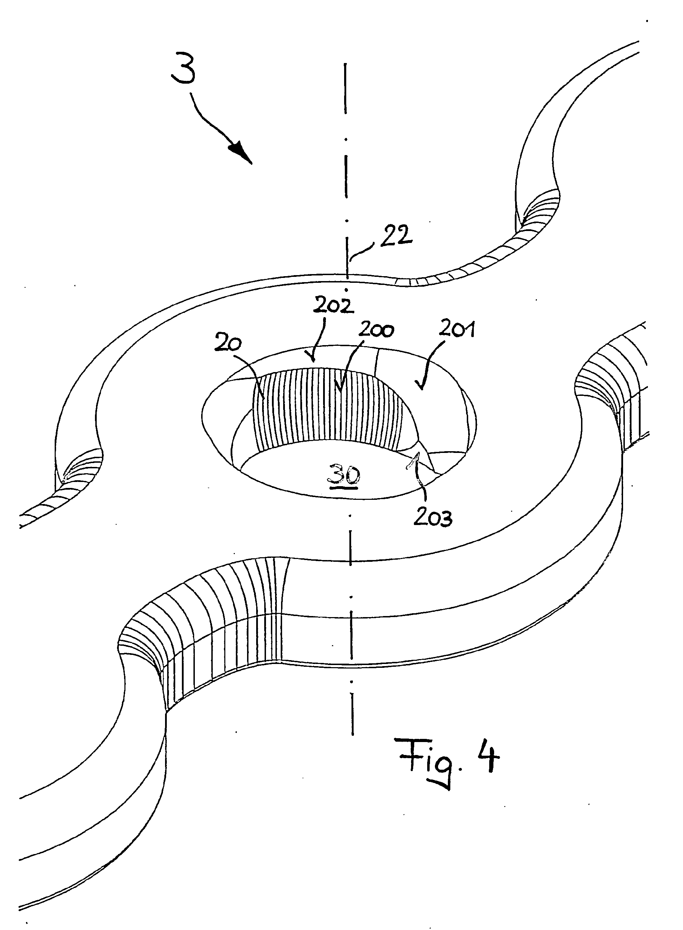

In **Fig. 3** **und** **Fig. 4** ist jeweils ein Ausschnitt aus einer Knochenplatte 3 in einer perspektivischen Ansicht dargestellt. Das Plattenloch 30 der Knochenplatte 3 ist mit einer Aufnahme 2 versehen, die einen Verblockungsteil eines Verblockungselements, also z.B. den Schraubenkopf 10 der Knochenschraube 1, aufnehmen kann. Die Aufnahme 2 weist auf ihrer umlaufenden Innenwand beim gezeigten Ausführungsbeispiel drei gleichmässig entlang des Umfangs verteilte Ausnehmungen 20 auf, bei denen sich die Innenwand 200 - in einer Azimutebene betrachtet, also in einer Ebene senkrecht zur Längsachse 22 der Aufnahme - keilförmig von der Längsachse der Aufnahme 2 (hier identisch mit der Plattenlochachse) nach aussen weg erweitert. Die Längsachse 22 verläuft hier senkrecht zur Plattenebene, grundsätzlich könnte die Längsachse aber auch unter einem anderen Winkel auf der Plattenebene stehen (schräges Plattenloch mit Aufnahme).

An der umlaufenden Innenwand erkennt man weiterhin eine Auslaufkontur 201, welche jeweils an die keilförmig sich erweiternde Innenwand 200 im Bereich der Ausnehmung 20 anschliesst. Diese Auslaufkontur 201 dient zum Ausleiten der Schraube 1, um sie einfacher wieder aus der Aufnahme 2 entnehmen zu können.

Ferner erkennt man eine Ansenkung 202, die zur Aufnahme eines Schraubenkopfs mit sphärischer Kopfunterseite einer konventionellen Knochenschraube dient, also einer Knochenschraube, bei welcher der Schraubenkopf nicht mit der Aufnahme im Plattenloch der Knochenplatte verblockt wird.

Der Bereich 203 der Innenwand der Aufnahme 20 ergibt sich aus der zylindrischen Kernbohrung des Plattenlochs 30, welche als erstes hergestellt wird, bevor die übrigen Bereich durch Fräsen (Bereich 200) bzw. Bohren (Bereich 203) und Senken (Bereich 202) hergestellt werden.

**Fig. 5 und Fig. 6** zeigt jeweils eine schematische Darstellung der Aussenkontur des Schraubenkopfes und der entsprechenden Innenkontur der Aufnahme in einer Azimutebene, einmal in unverblockter Position (Fig. 5) und einmal in verblockter Position (Fig. 6). Dort erkennt man auch etwas besser, dass die Aussenkontur der Klemmfläche 100 des Schraubenkopfs 10 in einer Azimutebene beispielsweise durch die oben bereits genannte "Wurzelfunktion" beschrieben werden kann, wobei in Fig. 5 insbesondere jeweils der Radius r am Anfang der durch die "Wurzelfunktion" beschriebenen Aussenkontur sowie der Azimutwinkel α dargestellt sind. Ebenfalls zu erkennen ist die sich gleichfalls hier entsprechend einer "Wurzelfunktion" erweiternde Fläche 200 der Ausnehmung 20 der Aufnahme. Verdreht man den Schraubenkopf gegenüber der Aufnahme im Uhrzeigersinn, so wird der Schraubenkopf in der Aufnahme verblockt und es resultiert die Situation, die in Fig. 6 dargestellt ist. Die Schraube (genauer gesagt: der Schraubenkopf) und die Knochenplatte sind fest miteinander verbunden.

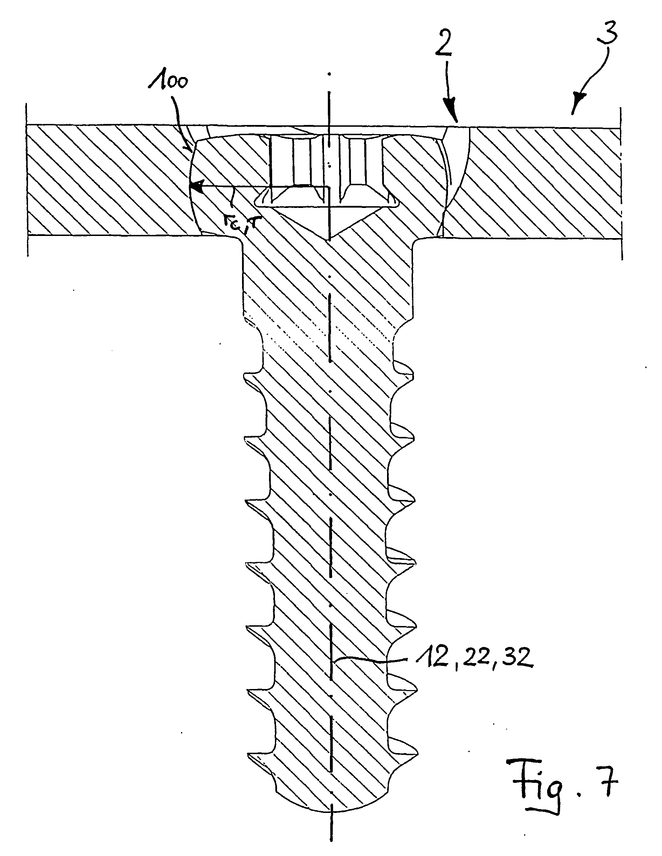

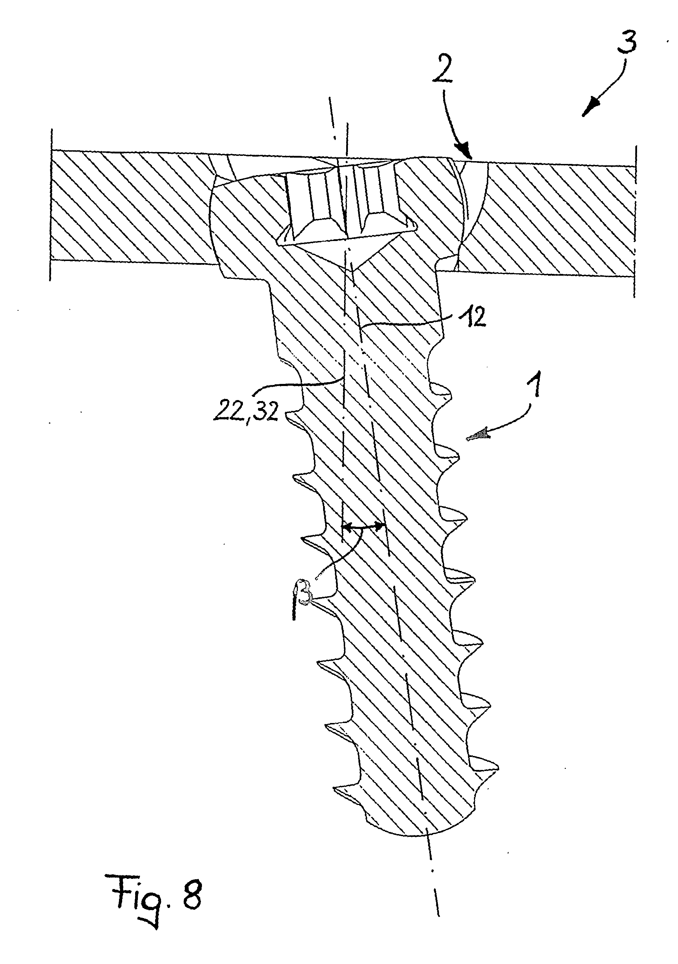

**Fig. 7** zeigt eine in der Aufnahme 2 verblockte Knochenschraube 1 im Längsschnitt (entsprechend der Situation in Fig. 6). Dabei fällt die Längsachse 12 der Schraube 1 mit der Längsachse 32 des Plattenlochs der Knochenplatte 3 bzw. mit der Längsachse 22 der Aufnahme zusammen, die Schraubenlängsachse und die Längsachse des Plattenlochs bzw. der Aufnahme schliessen keinen Winkel miteinander ein. Ferner erkennt man in Fig. 7 noch, dass der Krümmungsradius r_{c} der in Längsrichtung betrachtet sphärischen Klemmfläche 100 seinen Fusspunkt auf der Längsachse 12 der Schraube 1 hat analoge Betrachtungen gelten für die Ausnehmung. Dies bedeutet, dass das Verhältnis des Krümmungsradius r_{c} zum aktuellen Abstand r (siehe Fig. 5) des Punkts auf der Klemmfläche von der Längsachse 12 der Schraube hier genau 1 beträgt. Da der Krümmungsradius r_{c} jedoch konstant ist, der aktuelle Abstand r eines Punkts sich hingegen im Bereich der Klemmfläche sich in Umfangsrichtung ändert (die Klemmfläche - wie auch die Ausnehmung - erweitert sich von der Längsachse 12 bzw. 22 weg nach aussen), kann dies nicht über den gesamten Bereich der Klemmfläche bzw. Ausnehmung gelten. Andererseits muss der Krümmungsradius r_{c} innerhalb gewisser Grenzen liegen, damit eine Verkippung der Schraube einerseits, und eine sichere Verblockung der Schraube andererseits möglich ist. Als zweckmässig hat sich diesbezüglich ein Verhältnis des Krümmungsradius' r_{c} zum Abstand r des Punkts auf der Klemmfläche der Schraube bzw. der Innenwand der Ausnehmung von der Längsachse 12 bzw. 22 im Bereich von 0.3-3 erwiesen, vorzugsweise beträgt dieses Verhältnis ungefähr etwa 1. Dies gilt für die Innenwand der Aufnahme gleichermassen wie für die Aussenwand der Schraube. Anders als in Fig. 7 ist die Position der in **Fig. 8** gezeigten, ebenfalls in der Aufnahme 2 verblockten Knochenschraube 1. Dort schliessen die Längsachse 32 des Plattenlochs bzw. die Längsachse 22 der Aufnahme und die Schraubenlängsachse 12 einen Winkel β ein, wobei die Neigung der Schraube 1 nicht nur in Richtung der Längsachse der Knochenplatte 3 erfolgen kann, sondern in jeder beliebigen Richtung. Da sich die Schraube hier wiederum quasi in einem Kugelsitz befindet, ist die Verblockung erneut möglich.

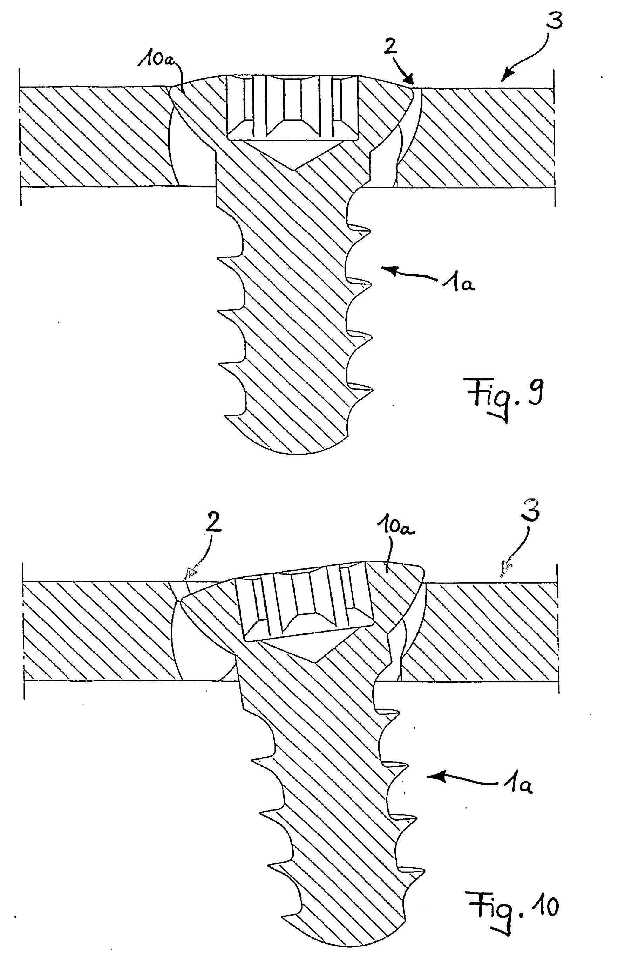

**Fig. 9 und Fig. 10** zeigen jeweils eine Knochenplatte 3, bei der eine konventionelle Knochenschraube 1a mit sphärischer Kopfunterseite einmal gerade (Fig. 9) und einmal unter einem Winkel (Fig. 10) von der Aufnahme 2 aufgenommen wird. Dabei liegt der Kopf 10a der Schraube 1a auf der entsprechenden Ansenkung 202 (siehe Fig. 3, Fig. 4) der Aufnahme 2 auf. Die Aufnahme 2 kann also auch konventionelle Schrauben 1a mit sphärischer Kopfunterseite aufnehmen, wodurch die Knochenplatte 3 in konventioneller Art und Weise gegen den Knochen gedrückt fixiert werden kann, zu einer Verblockung von Schraube 1a und Knochenplatte 3 bzw. Aufnahme 2 wie vorstehend beschrieben kommt es dabei jedoch nicht.

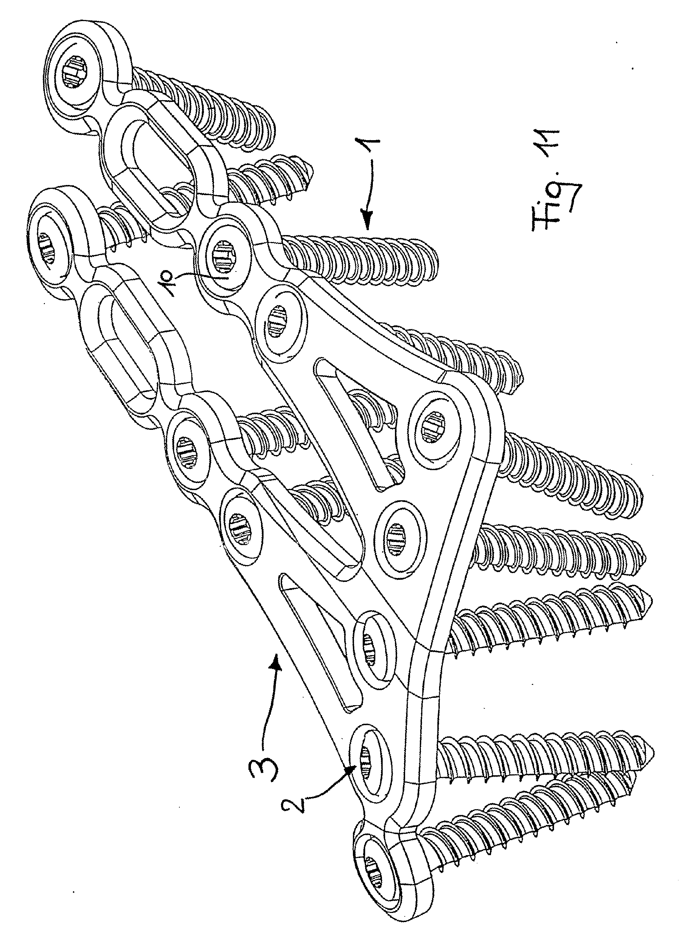

In **Fig. 11** ist eine Knochenplatte 3 dargestellt, wie sie im Bereich der Handchirurgie zum Einsatz kommen kann, mit mehreren in den Plattenlöchern unter verschiedenen Winkeln in der entsprechenden Aufnahme 2 angeordneten und verblockten Schraubenköpfen 10 von Knochenschrauben 1. Gerade im Bereich der Handchirurgie müssen häufiger mehrere kleine Knochensegmente zum Teil nur mit einer einzigen Schraube fixiert und relativ zueinander positioniert werden, und es ist dabei insbesondere auch wichtig, dass die Position eines bereits positionierten Knochensegments noch einmal ohne grossen Aufwand verändert werden kann, um eine optimale Versorgung zu erreichen.

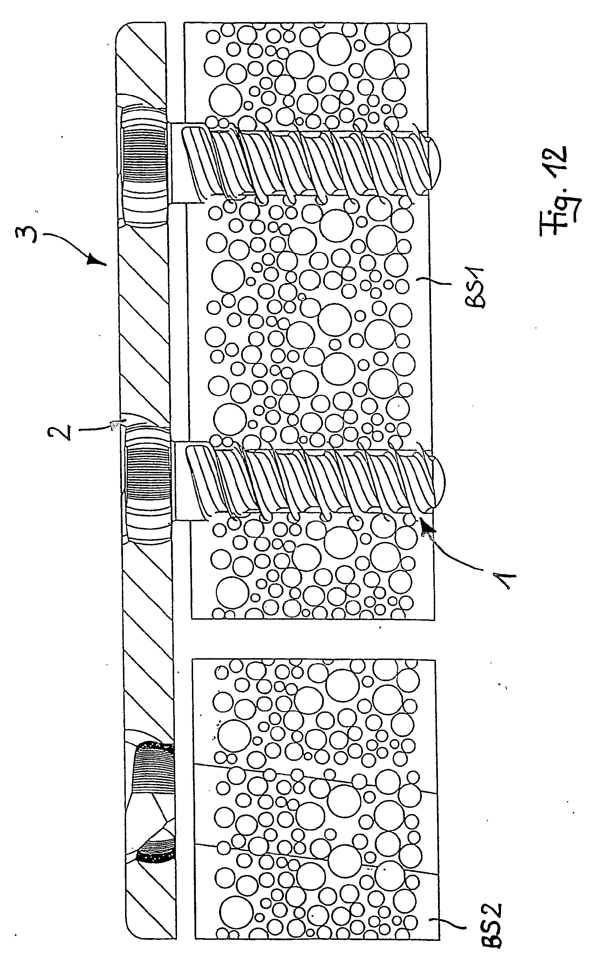

Dies soll im Folgenden mit Hilfe von **Fig. 12-19** etwas näher erläutert werden. Zu diesem Zweck sind zwei Knochensegmente BS1 und BS2 stark vereinfacht dargestellt, sowie eine Knochenplatte 3, die bereits mit Hilfe von zwei Schrauben 1 mit dem Knochensegment BS1 verbunden ist. Die beiden mit dem Knochensegment BS1 verbundenen Schrauben 1 sind dabei in der zuvor erläuterten Weise in der Aufnahme 2 der Knochenplatte 3 verblockt, wobei die Ausrichtung der Schraubenachse nicht unbedingt senkrecht zum Knochen sein muss. Ausserdem erfolgt die Beschreibung mit Hilfe von Plattenlöchern, deren Achse senkrecht durch die Knochenplatte hindurch verläuft. Es ist jedoch auch denkbar, dass die Achse der Plattenlöcher bzw. die Längsachse der Aufnahme unter einem von 90° verschiedenen Winkel durch die Knochenplatte hindurch verläuft (Schrägbohrung) - das Funktionsprinzip bleibt grundsätzlich gleich.

In **Fig. 12** ist dabei in das Knochensegment BS2 noch keine Schraube 1 eingeschraubt, es kommt lediglich ein Plattenloch mit einer entsprechenden Aufnahme 2 über dem Ort zu liegen, wo eine Schraube 1 in das Knochensegment BS2 eingeschraubt werden wird. Angedeutet ist jedoch bereits, dass das Einbringen der Schraube 1 unter einem Winkel relativ zu der Senkrechten erfolgen soll.

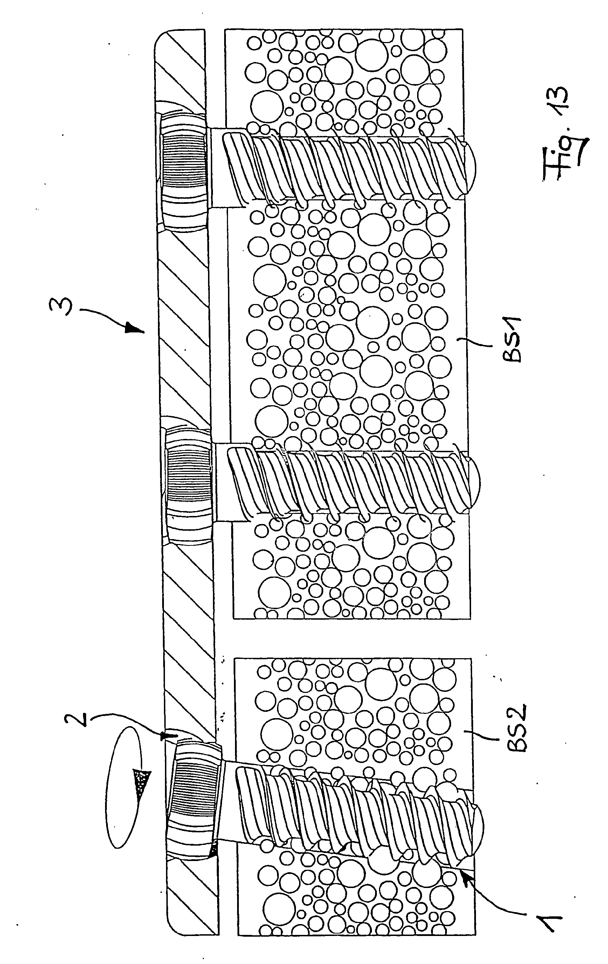

In **Fig. 13** ist die Knochenschraube 1 dann in das Knochensegment BS2 eingeschraubt und in der Aufnahme 2 der Knochenplatte 3 verblockt. Dazu wird die Schraube 1 in der Richtung (Uhrzeigersinn) gedreht, die durch den oberhalb der entsprechenden Aufnahme 2 angedeuteten Pfeil ersichtlich ist.

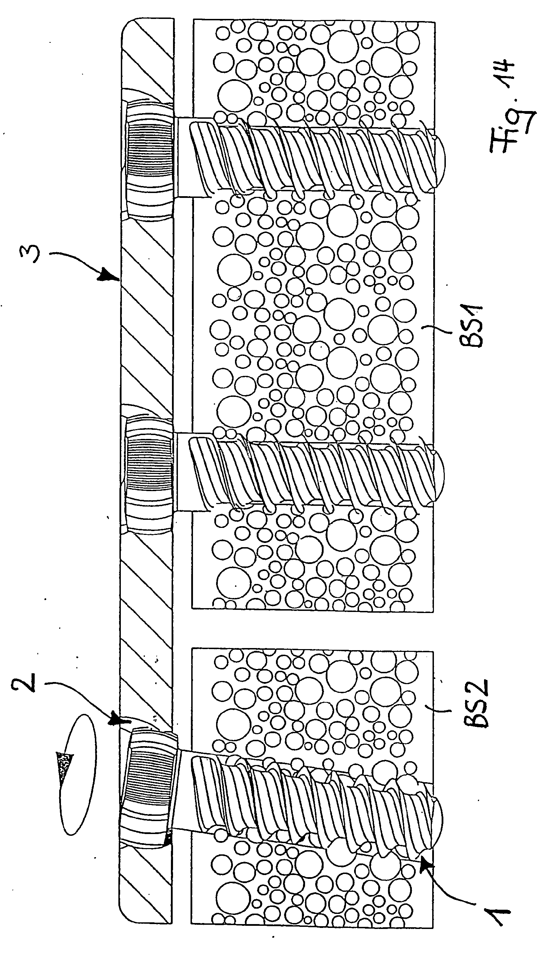

Wenn nun das Knochensegment BS2 neu relativ zum Knochensegment BS1 positioniert werden soll, muss die Verblockung der in das Knochensegment BS2 eingeschraubten Schraube 1 wieder gelöst werden. Dies erfolgt durch Drehen der Schraube 1 in Richtung (Gegenuhrzeigersinn) des oberhalb der entsprechenden Aufnahme angedeuteten Pfeils **(****Fig. 14****).**

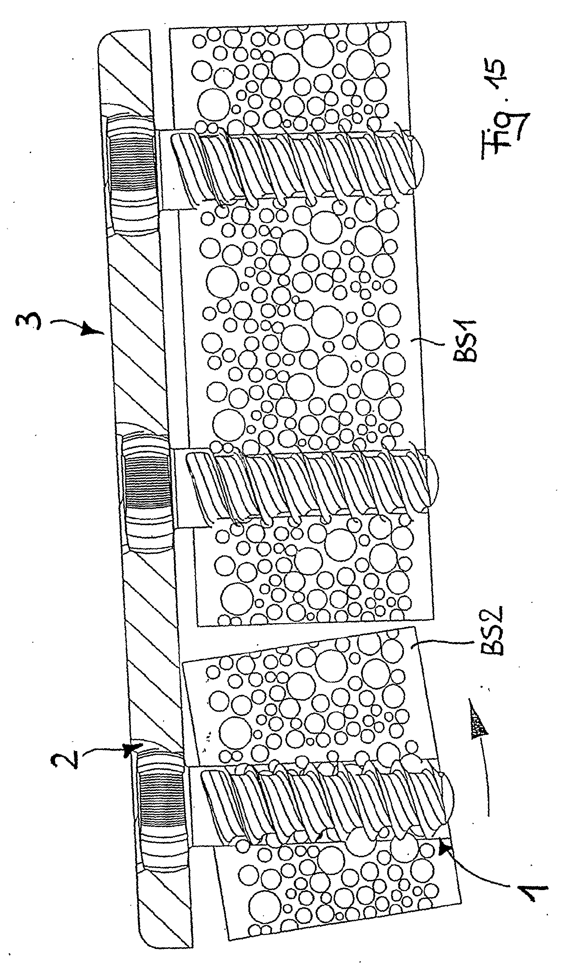

Nach dem Lösen der Verblockung kann das Knochensegment BS2 mit der darin eingeschraubten Schraube 1 z.B. um einen Winkel in eine gewünschte Position gekippt werden, wie in **Fig. 15** durch den Pfeil unterhalb des Knochensegments BS2 angedeutet.

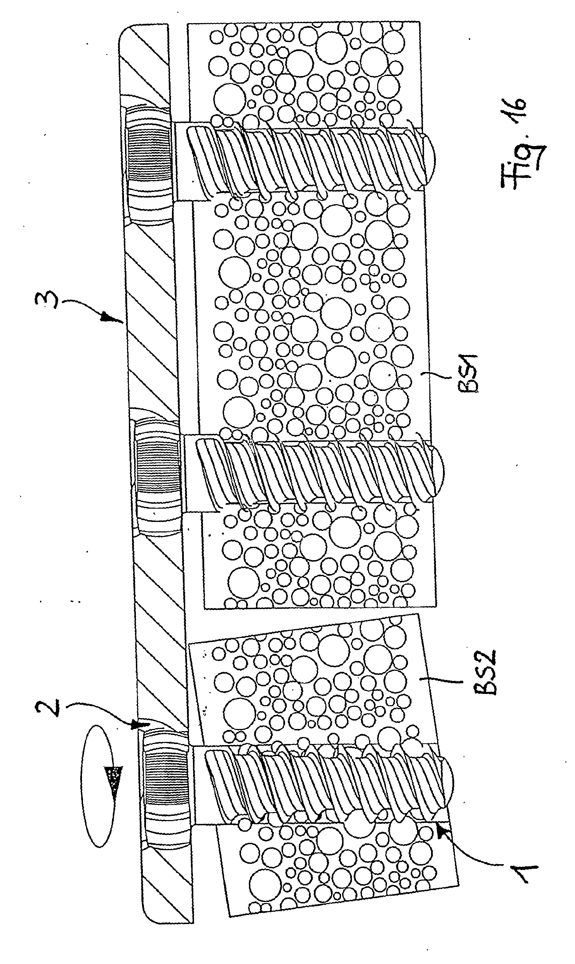

Nach dem Verkippen des Knochensegments BS2 in die neue gewünschte Position erfolgt erneut eine Verblockung durch Drehen der Schraube 1 in Richtung (Uhrzeigersinn) des Pfeils, der in **Fig. 16** oberhalb der Schraube 1 angedeutet ist.

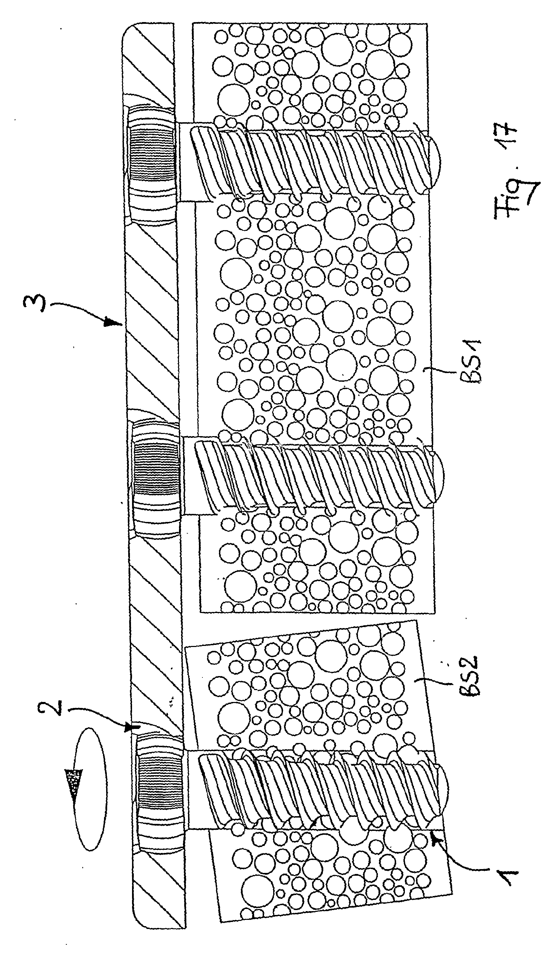

Stellt sich erneut heraus, dass die Position des Knochensegments BS2 geändert werden soll, wird die Verblockung erneut gelöst durch Drehen der Schraube 1 in Richtung des Pfeils, der in **Fig. 17** oberhalb der Schraube 1 angedeutet ist (Gegenuhrzeigersinn).

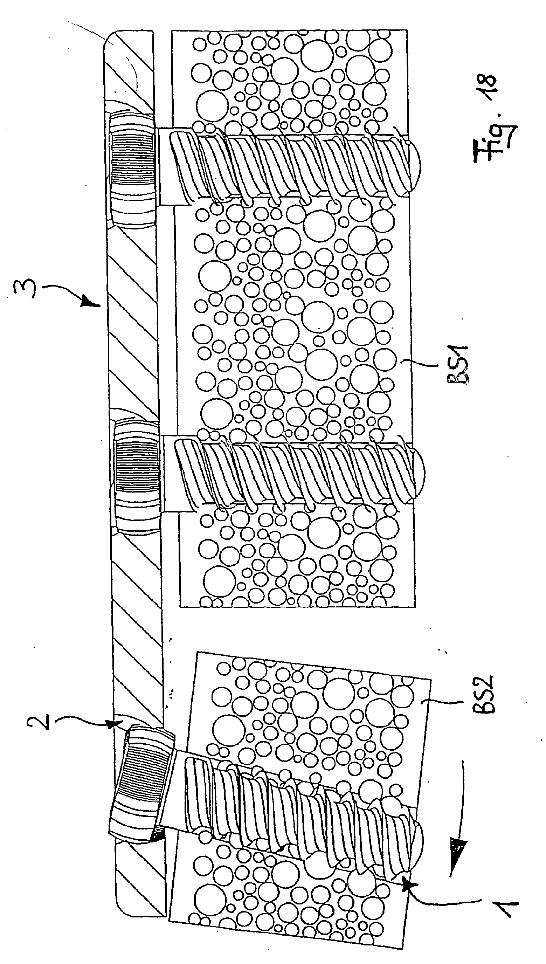

Sodann kann das Knochensegment in die neue gewünschte Position gebracht werden, beispielsweise derart verkippt werden, wie es in **Fig. 18** durch den Pfeil unterhalb des Knochensegments BS2 angedeutet ist.

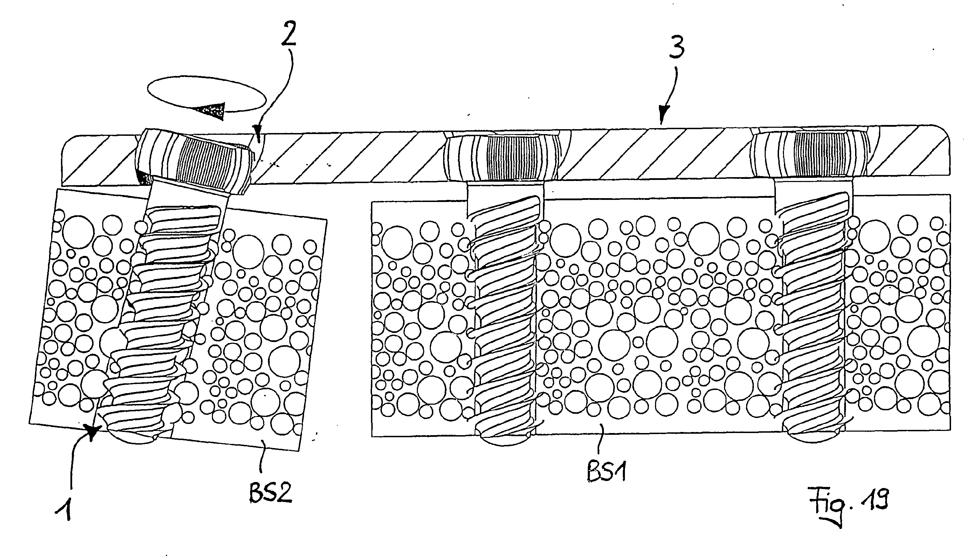

Zum erneuten Verblocken (Fixieren) wird die Schraube 1 in Richtung des in **Fig. 19** angedeuteten Pfeils gedreht (Uhrzeigersinn), wodurch die Relativstellung der Knochensegmente BS1 und BS2 zueinander festgelegt wird. Im Frakturspalt kann sich dann mit der Zeit neuer Knochen bilden.

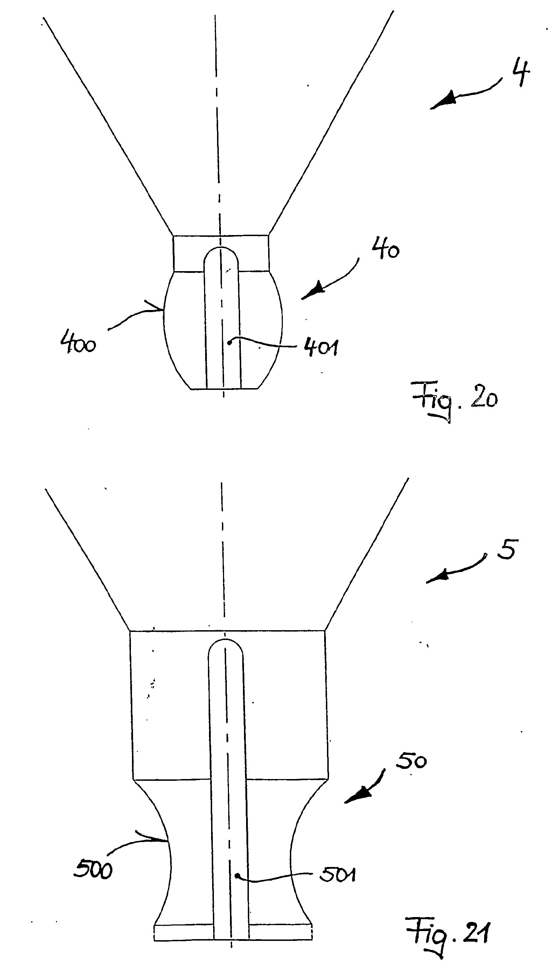

In **Fig. 20** ist - in schematischer Darstellung - ein Ausschnitt eines Ausführungsbeispiels eines Fräsers 4 gezeigt, mit welchem die Innenwand 200 der Ausnehmung 20 hergestellt werden kann. Man erkennt, dass der Fräser einen konvexen Fräserkopf 40 mit einer zumindest annähernd sphärisch ausgebildeten Kontur 400 aufweist, mit welcher die Innenwand 200 der Ausnehmung 20 hergestellt werden kann. Dazu wird der Fräser 4 beispielsweise CNC-gesteuert entsprechend der "Wurzelfunktion" oder entsprechend der "logarithmischen Spirale" (oder der Kreisbahn, Evolvente, etc., siehe oben) durch den Bereich der jeweiligen Ausnehmung 20 geführt, sodass die gewünschte Kontur der Innenwand 200 in diesem Bereich entsteht. Zur Abführung des Spans dient die im Fräserkopf 40 vorgesehene Nut 401. Zuvor ist zunächst eine zylindrische Kernbohrung durch die Knochenplatte 3 gebohrt worden (Kernbohrung des Plattenlochs 30), in welche der Fräser 4 eingeführt werden kann, um dann die entsprechende Kontur der Innenwand 200 CNC-gesteuert herzustellen.

Die Aussenfläche des Schraubenkopfs 10 lässt sich mit einem Fräser 5 mit einem konkaven Fräserkopf 50 mit einer ebenfalls zumindest annähernd sphärischen ausgebildeten Kontur 500 herstellen, wie er in **Fig. 21** schematisch dargestellt ist. Die Nut 501 dient der Abführung des Spans. Zur Herstellung der Aussenfläche des Schaubenkopfs 10 wird der Fräser 5 entlang eines z.B. zylindrischen Schraubenkopfs geführt, z.B. CNC-gesteuert. Im Bereich der Klemmflächen 100 des Schraubenkopfs 10 kann dies z.B. entsprechend der "Wurzelfunktion" erfolgen oder z.B. entsprechend der "logarithmischen Spirale" (oder der Kreisbahn, Evolvente, etc., siehe oben). In den Bereichen zwischen den Klemmflächen kann dies nach einer anderen Funktion erfolgen, hier muss im Grunde nur sichergestellt sein, dass beim Verkippen der Schraube die Aussenflächen des Schraubenkopfs 10 nicht mit der Innenwand der Aufnahme 2 kollidieren und eine Verkippung verhindern kann. Ansonsten kann in Längsrichtung der Schraube betrachtet auch in den Bereichen zwischen den Klemmflächen 100 die Aussenfläche des Schraubenkopfs mit dem gleichen Fräser 5 hergestellt werden wie die Klemmflächen 100.

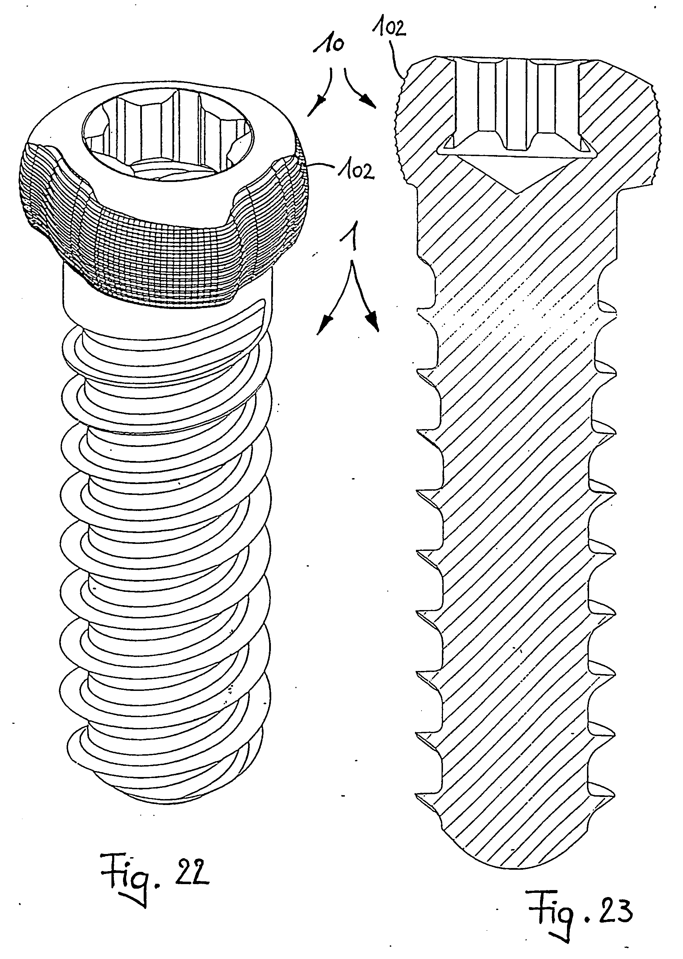

In **Fig. 22** und **Fig. 23** ist in einer perspektivischen Ansicht bzw. im Längsschnitt durch die Schraube 1 dargestellt, dass die Aussenfläche des Schraubenkopfs 10 der Schraube 1 noch mit einer Struktur 102 versehen sein kann. Eine solche zusätzliche (Fein-)Struktur 102 - die hier beispielsweise in Form von umlaufenden Rillen ausgeführt ist - kann die Verblockung des Schraubenkopfs 10 in der Ausnehmung 20 noch weiter verbessern.

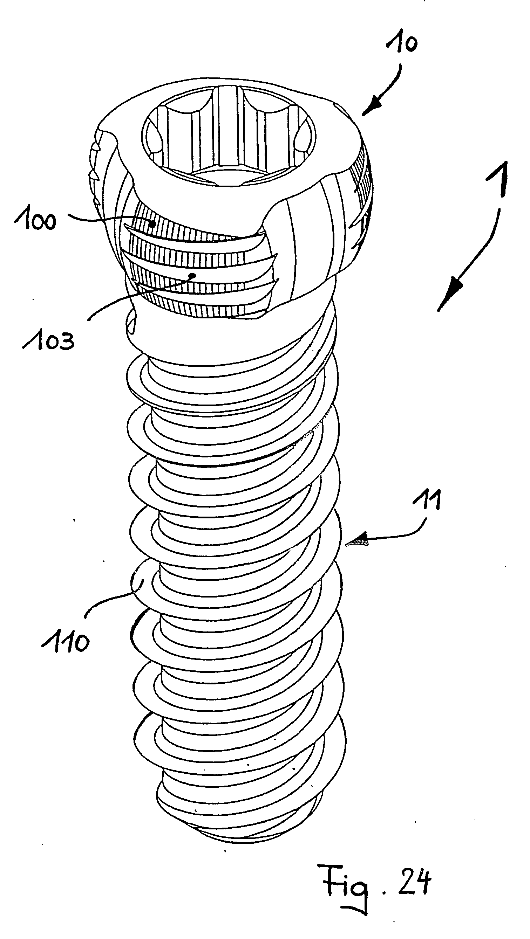

In **Fig. 24** ist in einer perspektivischen Ansicht ein Ausführungsbeispiel der Schraube 1 dargestellt, bei der im Schraubenkopf 10 in der Klemmfläche 100 rillenförmige Vertiefungen 103 vorgesehen sind. Diese rillenförmigen Vertiefungen verlaufen im wesentlichen in der gleichen Richtung und im wesentlichen mit der gleichen Steigung wie das Schaftgewinde 110 am Schraubenschaft. Diese rillenförmigen Vertiefungen 103 können beim Eindrehen der Schraube 1 eine wesentliche Rolle spielen, wie im folgenden erläutert wird.

Dazu stelle man sich vor, dass der Schraubenschaft der Schraube aus Fig. 24 durch das Plattenloch 30 hindurch in den Knochen eines Patienten eingeschraubt und der Schraubenkopf 10 in der Aufnahme 2 der Knochenplatte 3 (Fig.3) fixiert (verblockt) werden soll. Gegen Ende des Einschraubens, also dann, wenn der Schraubenschaft 11 weitestgehend in den Knochen eingeschraubt ist, taucht der Schraubenkopf 10 in die Aufnahme 2 der Knochenplatte 3 ein. Da man nicht voraussagen kann, in welcher azimutalen Relativposition das Eintauchen des Schraubenkopfs 10 in die Aufnahme 11 erfolgt, kann es zu Situationen kommen, in denen die Klemmfläche 100 mit einem nach innen vorstehenden Bereich der Aufnahme 2 kollidiert, der durch die Kontur der Innenwand 200 der Aufnahme 2 bedingt beispielsweise dort vorliegt, wo die Bereiche 203 (Kernbohrung des Plattenlochs 30) und die Ein-/Auslaufkontur 201 aneinander angrenzen.

Würde man in einem solchen Falle die Schraube aus Fig. 1 noch weiter einschrauben, könnte die Klemmfläche 100 diesen nach innen vorstehenden Bereich bis zu einem durch das Material (Titan) begrenzten Ausmass verformen, wobei sich dann das Material nicht mehr weiter verformen lässt und der Schraubenkopf 10 dann an dieser Stelle verklemmt würde. Die Verklemmung fände aber nicht dort statt, wo sie eigentlich erwünscht ist, nämlich zwischen der Klemmfläche 100 und der sich nach aussen erweiternden Ausnehmung 20 der Innenwand 200, und folglich würde es sich bei dieser Verklemmung nicht um die erwünschte flächige Verblockung handeln. Ausserdem würde in diesem Bereich die Kontur der Aufnahme und/oder die Klemmfläche 100 des Schraubenkopfs 10 beschädigt werden können, weshalb in einem solchen Fall die Verbindung zwischen Schraube 1 und Knochenplatte 3 weniger stabil sein kann als bei der erwünschten Verblockung. Darüber hinaus ist es möglich, dass das bereits anhand der Fig. 12-19 beschriebene Lösen, Umpositionieren und Wiederfixieren (Verblocken) des Knochenfragments wegen einer zuvor eingetretenen Beschädigung der Schraube 1 und/oder der Aufnahme 2 der Knochenplatte 3 nicht mehr möglich ist. Für den behandelnden Arzt ist es dagegen schwierig wenn nicht gar unmöglich festzustellen, ob die Verklemmung nun eine reguläre - erwünschte - Verblockung des Schraubenkopfs 10 in der Aufnahme 2 ist oder die zuvor beschriebene ungewollte Verklemmung.

Damit selbst in den seltenen Fällen, in denen der vorstehend beschriebene Fall auftritt, eine reguläre Verblockung des Schraubenkopfs 10 in der Aufnahme 2 der Knochenplatte 3 erfolgen kann, sind in der Klemmfläche 100 rillenförmige Vertiefungen 103 vorgesehen. Die rillenförmigen Vertiefungen 103 verlaufen dabei - wie oben bereits erwähnt - im wesentlichen in der gleichen Richtung und im wesentlichen mit der gleichen Steigung wie das Schaftgewinde 110. Das ist deshalb so, weil beim weiteren Einschrauben der Schraube 1 der Schraubenkopf 10 und damit auch die Klemmfläche 100 entsprechend der Steigung des Schaftgewindes sowohl in azimutaler als auch in axialer Richtung bewegt wird. Die ungewollte Verklemmung, wie sie oben beschrieben ist, wird nun dadurch verhindert, dass der nach innen vorspringende Bereich der Aufnahme 2 in eine der rillenförmigen Vertiefungen 103 hineingleiten kann. Durch den Verlauf und die Steigung der rillenförmigen Vertiefungen 103 wird der nach innen vorspringende Bereich dann von der rillenförmigen Vertiefung 103, in die er hinein geglitten ist, durchfurcht, weil das weitere Einschrauben der Schraube 1 bewirkt, dass die axiale und azimutale Bewegung des Schraubenkopfs 10 stets in gleichem Ausmass erfolgt, mit welchem das Schaftgewinde 110 in den Knochen eindringt. Nach dieser Durchfurchung läuft die Schraube wieder frei, d.h. es besteht kurzzeitig kein Kontakt zwischen Aufnahme und Schraubenkopf. Beim weiteren Einschrauben tritt dann die zuvor bereits beschriebene Verblockung ein. Trotz des Vorhandenseins der rillenförmigen Vertiefungen 103 verbleibt eine ausreichend grosse Klemmfläche 100, um eine gute Verblockung des Schraubenkopfs 10 in der Aufnahme 2 der Knochenplatte 3 zu bewirken.

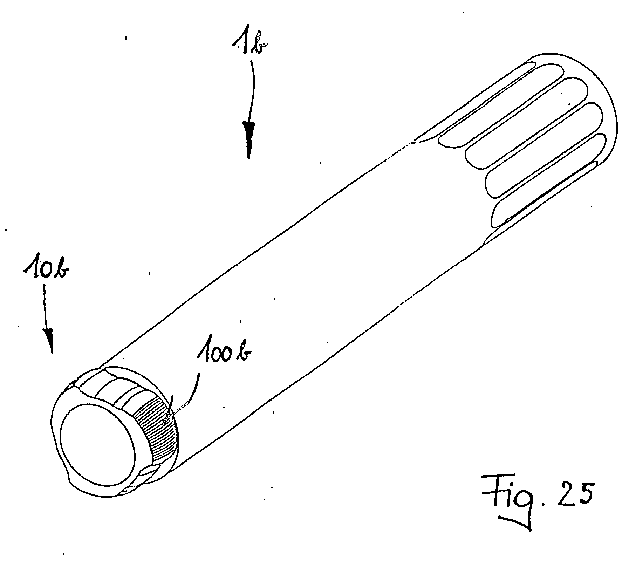

**Fig. 25** zeigt ein weiteres Ausführungsbeispiel eines Verblockungselements in Form einer Bohrführung 1b. Die Bohrführung 1b ist mit einem Verblockungsteil 10b versehen, welches ähnlich ausgebildet ist wie der Schraubenkopf 10 der Knochenschraube 1 und daher insbesondere auch unter verschiedenen Winkeln relativ zur Längsachse der Aufnahme bzw. des Plattenlochs - verblockt werden kann. Dazu ist der Verblockungsteil 10b mit Klemmflächen 100b versehen, die in gleicher Weise wie bereits oben anhand der Klemmflächen 100 des Schraubenkopfs 10 in der Aufnahme 2 verblockt werden können. Durch die verblockte Bohrführung kann dann beispielsweise ein Bohrer geführt werden, mit dem im Knochen eine Bohrung unter einem entsprechenden Winkel (der eben durch die Anordnung der Bohrlehre frei wählbar ist) für eine Knochenschraube hergestellt wird.

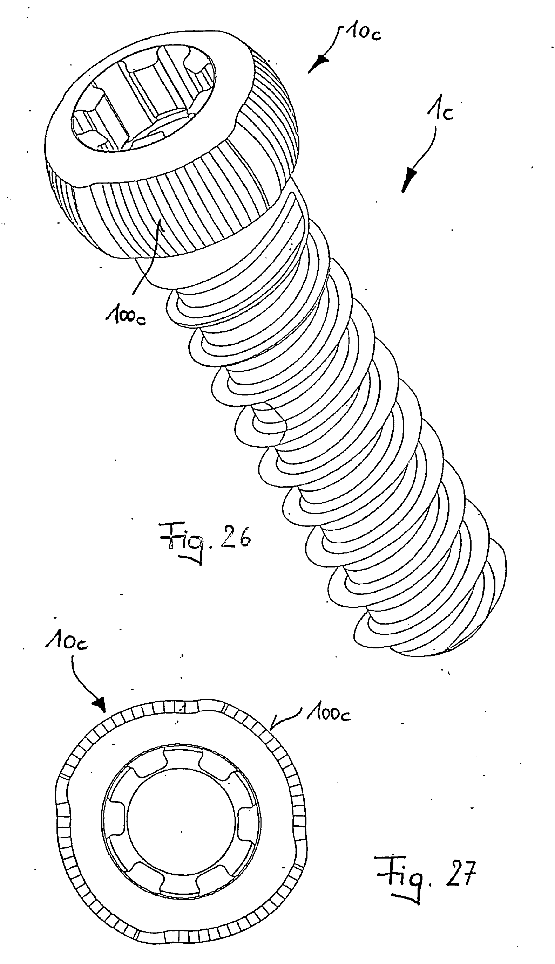

In **Fig. 26** ist ein weiteres Ausführungsbeispiel eines Verblockungselements in Form einer weiteren Knochenschraube 1c dargestellt, welche sich von der Knochenschraube 1 im wesentlichen dadurch unterscheidet, dass der Schraubenkopf 10c nicht drei, sondern vier gleichmässig über den Umfang verteilte Klemmbereiche 100c aufweist. Dies ist auch aus der Aufsicht in **Fig. 27** gut ersichtlich. Die zugehörige Aufnahme (nicht dargestellt), weist dann vorzugsweise ebenfalls vier Ausnehmungen aus zum Verblocken der Schraube 1c in der zugehörigen Aufnahme.

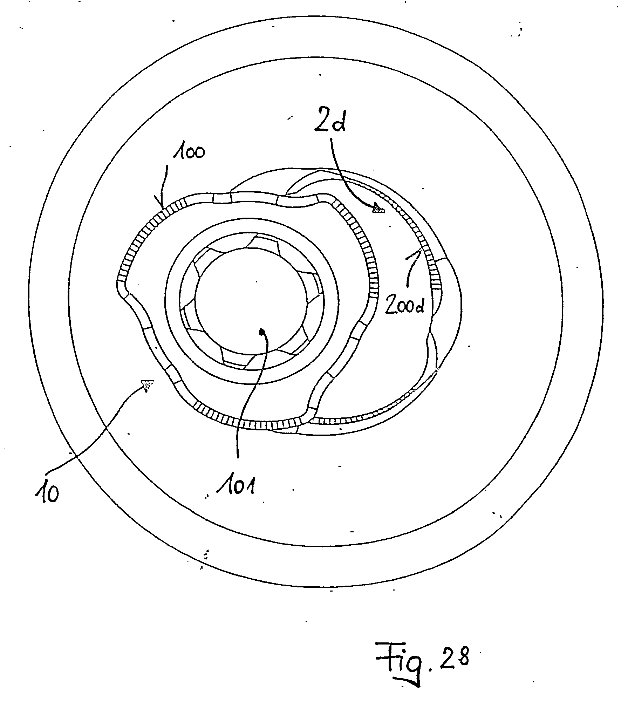

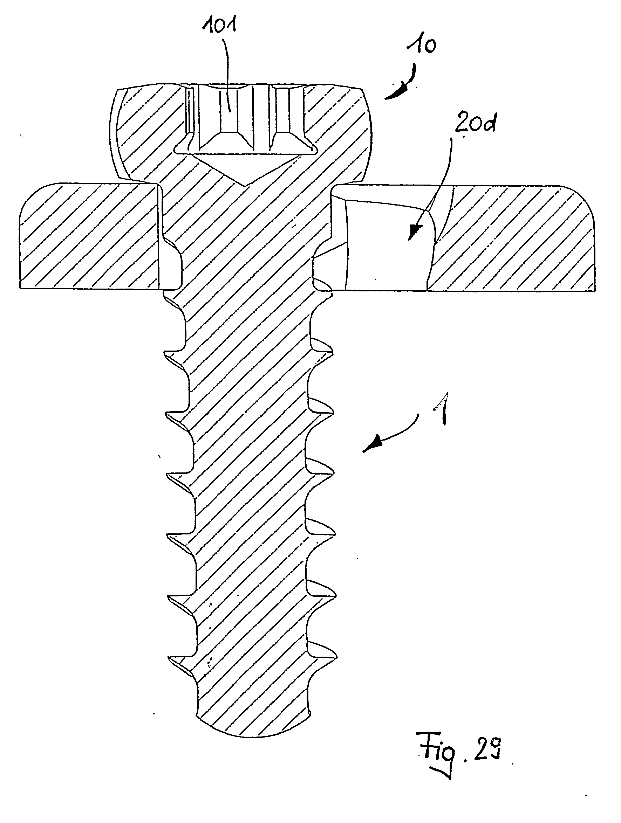

In **Fig. 28** ist eine Aufsicht auf ein weiteres Ausführungsbeispiel einer Aufnahme 2d in einem Langloch 30d einer Knochenplatte gezeigt, die z.B. bei der Kompressionsosteosynthese verwendet werden kann. **Fig. 29** zeigt eine entsprechende Darstellung im Längsschnitt. Man erkennt zudem die zugehörige Knochenschraube 1 in unverblockter Position zu Beginn der Kompression. In der Aufnahme 2d sind ebenfalls Ausnehmungen 20d vorgesehen, deren Innenwand 200d grundsätzlich ähnlich ausgebildet sein können wie die jeweilige Innenwand 200 der Ausnehmungen 20, die bereits weiter oben erläutert wurde. Ebenso ist die Schraube 1 mit Klemmflächen 100 am Schraubenkopf 10 versehen.

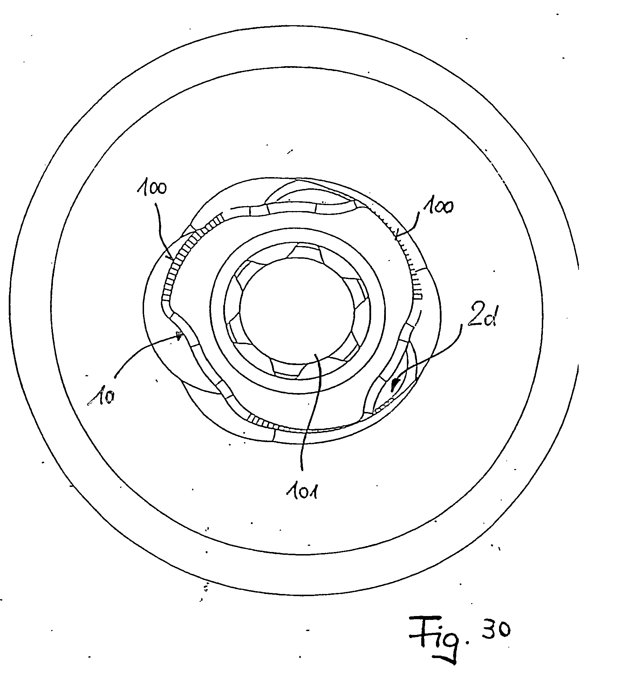

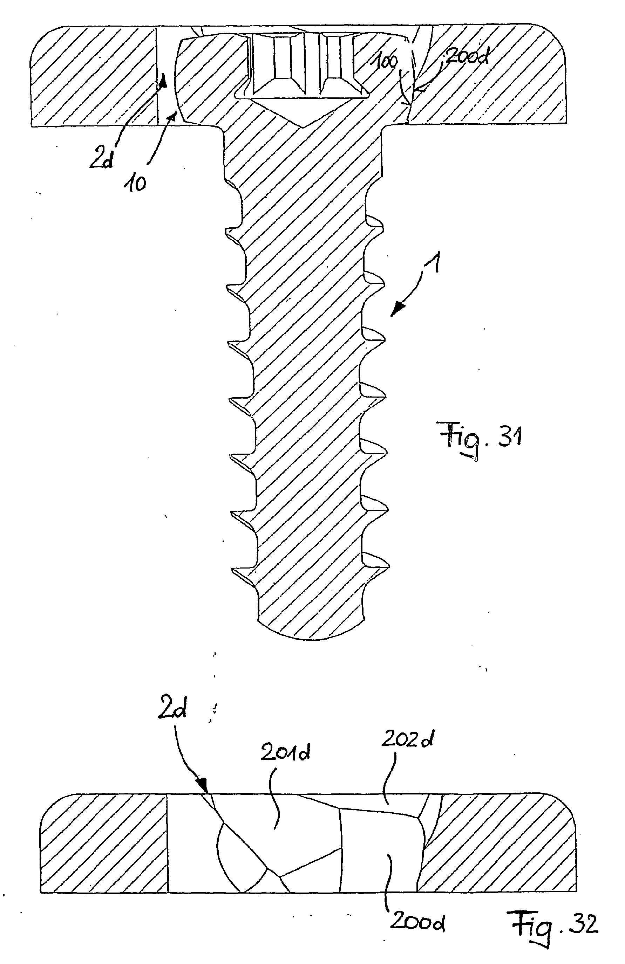

Bei einer Kompressionsosteosynthese kann beispielsweise wie folgt vorgegangen werden: Die Knochenschraube 1 ist bereits in eines von zwei Knochensegmenten diesseits und jenseits einer Fraktur eingeschraubt, und das andere Knochensegment ist mittels einer Knochenschraube bereits fest mit der Knochenplatte verbunden. Nun gleitet durch Drehen der Knochenschraube (Eingriff eines Werkzeugs in die Eingriffskontur 101) die Schraube 1 in die Aufnahme 2d hinein. Dabei wird das mit der Schraube 1d verbundenen Knochensegment gegen das bereits fest mittels einer Schraube mit der Platte verbundene Knochensegment gedrückt. In dieser Position wird in der bereits weiter oben beschriebenen Art und Weise die Schraube 1 in der Aufnahme 2d verblockt. Diese Situation ist in der Aufsicht gemäss **Fig. 30** bzw. in dem Längsschnitt gemäss **Fig. 31** noch einmal dargestellt.

**Fig. 32** zeigt schliesslich noch einmal einen Längsschnitt durch die Aufnahme 2d des Langlochs. Man erkennt ausser der Innenwand 200d zur Verblockung mit der Klemmfläche 100 der Knochenschraube 1 (hier nicht dargestellt) noch die Ausleitfläche 201d, deren Funktion ebenfalls weiter oben bereits beschrieben ist (Ausleiten einer Schraube nach dem Lösen der Verblockung) sowie die Ansenkung 202d zur Aufnahme einer konventionellen Knochenschraube, mit der natürlich ebenfalls in konventioneller Weise eine Kompressionsosteosynthese (allerdings ohne Verblockung) möglich ist.

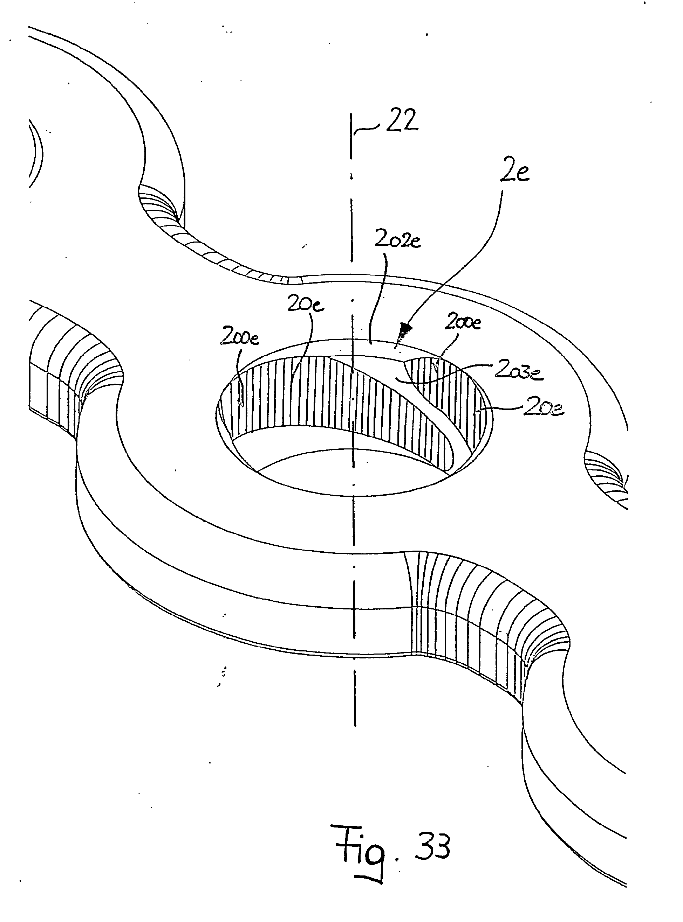

**Fig. 33** zeigt einen Ausschnitt aus einer Knochenplatte mit einem weiteren Ausführungsbeispiel einer erfindungsgemässen Aufnahme 2e im Plattenloch in einer perspektivischen Ansicht. Bei diesem Ausführungsbeispiel der Aufnahme 2e verläuft die Ausnehmung 20e nicht in einer zur Längsachse 22 der Aufnahme senkrechten Richtung (also nicht horzizontal) um, wie bei dem Ausführungsbeispiel gemäss Fig. 3 und Fig. 4, sondern läuft nach Art einer räumlichen Spirale um die Längsachse um. Ausser der Innenwand 200e im Bereich der Ausnehmung 20e erkennt man in Fig. 33 noch die sphärische Ansenkung 202e zur Aufnahme eines Schraubenkopfs einer konventionellen Knochenschrauben mit sphärischer Kopfunterseite, sowie den Bereich 203e der zylindrischen Kernbohrung. Eine Auslaufkontur ist bei dem gezeigten Ausführungsbeispiel nicht vorgesehen, könnte aber prinzipiell ebenfalls vorgesehen werden, z.B. wenn die räumliche Spirale nur eine sehr geringfügige Steigung aufweist, welche ein einfaches Entnehmen der Schraube nicht ermöglicht.

Die zugehörige Schraube bleibt hingegen so, wie sie bereits weiter oben beschrieben ist. Beim Eindrehen der Schraube wird der Schraubenkopf also nicht entlang der Ausnehmung in horizontaler Richtung geführt - wie dies bei dem Ausführungsbeispiel gemäss Fig. 3 und Fig. 4 der Fall ist - sondern entlang dem räumlich spiralförmigen Verlauf der Ausnehmung. Die Verkippbarkeit der Schraube ist hier ebenfalls gegeben und die Verblockung erfolgt in gleicher Weise wie bereits beschrieben. Die vorstehende Beschreibung betrifft beispielhaft die Verblockung von Knochenschrauben in Aufnahmen von entsprechenden Knochenplatten.

Es sei noch einmal angemerkt, dass die vorliegende Erfindung nicht auf dieses Anwendungsgebiet beschränkt ist, sondern allgemein eine Verblockung eines Verblockungselements in einer entsprechenden Aufnahme betrifft, wie dies auch in anderen Anwendungsgebieten durchaus - wie einleitend bereits diskutiert - erwünscht sein kann.

## Patentansprüche

1. *Aufnahmeelement, das eine Aufnahme* (2;2d;2e) für ein Verblockungselement (1;1b;1c) *aufweist*, mit einer umlaufenden Innenwand (200;200d;200e), die sich im wesentlichen in Richtung der Längsachse (22) der Aufnahme erstreckt und *die* mindestens eine Ausnehmung (20;20d;20e) aufweist, *in deren Bereich sich der Abstand der Innenwand in Abhängigkeit vom Umlaufwinkel um die Längsachse (22)* nach aussen von der Längsachse (22) weg *vergrössert*, um einen Verblockungsteil (10;10b;10c) des Verblockungselements mit einer entsprechenden Aussenkontur (100;100b;100c) aufnehmen und verblocken zu können, **dadurch gekennzeichnet, dass** die umlaufende Innenwand im Bereich der Ausnehmung (20;20d;20e) zumindest annähernd sphärisch, *paraboloidisch, ellipsoidisch* oder *hyperboloidisch* ausgebildet ist und beim Verblocken eine radiale Verklemmung zwischen der Ausnehmung (20;20d;20e) und der Aussenkontur (100;100b;100c) des Verblockungsteils (10;10b;10c) des Verblockungselements bewirkt.

2. *Aufnahmeelement* nach Anspruch 1, bei welcher die Ausnehmung (20;20d) so angeordnet ist, dass sie in einer Richtung senkrecht zur Längsachse (22) der Aufnahme umläuft.

3. *Aufnahmeelement* nach Anspruch 1, bei welcher die Ausnehmung (20e) so angeordnet ist, dass sie nach Art einer räumlichen Spirale um die Längsachse (22) der Aufnahme umläuft.

4. *Aufnahmeelement* nach einem der Ansprüche 1 bis 3, bei welcher die umlaufende Innenwand drei gleichmässig entlang ihres Umfangs verteilte Ausnehmungen (20;20d;20e) aufweist.

5. *Aufnahmeelement* nach einem der Ansprüche 1 bis 4, bei welcher die Kontur der Innenwand (200;200d;200e) im Bereich der Ausnehmung (20;20d;20e) bzw. Ausnehmungen durch einen Teil einer Funktion vom Typ r = a₁ + b₁√α beschrieben wird, wobei r den jeweiligen Abstand der Innenwand (200;200d;200e) von der Längsachse (22) der Aufnahme bedeutet, a₁ und b₁ Konstanten sind, und α für den jeweiligen Umlaufwinkel steht.

6. *Aufnahmeelement* nach einem der Ansprüche 1 bis 4, bei welcher die Kontur der Innenwand (200;200d;200e) im Bereich der Ausnehmung (20;20d;20e) bzw. Ausnehmungen durch einen Teil einer logarithmischen Spirale, durch einen Teil einer Kreisbahn, durch einen Teil einer Evolvente, oder ähnlich, beschrieben wird.

7. *Aufnahmeelement* nach einem der vorangehenden Ansprüche, bei welcher die Aufnahme (2;2d) an ihrer umlaufenden Innenwand eine in Umlaufrichtung an die Ausnehmung (20;20d) bzw. Ausnehmungen anschliessende Auslaufkontur (201;201d) aufweist zum Ausleiten des Verblockungselements (1;1b;1c) aus der Aufnahme.

8. *Aufnahmeelement* nach einem der vorangehenden Ansprüche, bei *welchem* die Aufnahme (2;2d;2e) mit einer Ansenkung (202;202d;202e) versehen ist zur Aufnahme eines Schraubenkopfs (10a) mit sphärischer Kopfunterseite.

9. Knochenplatte (3) mit Plattenlöchern (30), wobei mindestens ein Plattenloch (30) mit einer Aufnahme (2;2d;2e) versehen ist für ein Verblockungselement (1;1b;1c), welches mit der Knochenplatte (3) zu verblocken ist, z.B. für eine Knochenschraube (1;1c), für eine Bohrführung (1b) oder für einen Fixierstift, **dadurch gekennzeichnet, dass** die Aufnahme (2;2d;2e) *in einem Aufnahmeelement* nach einem der vorangehenden Ansprüche ausgebildet ist.

10. Knochenplatte (3) nach Anspruch 9, bei welcher mindestens ein Plattenloch als Langloch ausgebildet ist, in welchem die Aufnahme *in einem Aufnahmeelement* nach einem der Ansprüche *1 bis 8* ausgebildet ist.

11. Verblockungselement (1;1b;1c) zum Einbringen in *eine Aufnahme* (2;2d;2e) *eines Aufnahmeelements* und zum Verblocken mit dieser Aufnahme (2;2d;2e), bei welchem Verblockungselement (1;1b;1c) ein Verblockungsteil (10;10b;10c), z.B. ein Kopf, mit einer umlaufenden Aussenfläche versehen ist, die sich im wesentlichen in Richtung einer Längsachse (12) erstreckt und mindestens eine Klemmfläche (100;100b;100c) aufweist, *in deren Bereich* sich - in einer Azimutebene senkrecht zur Längsachse (12) betrachtet - *der Abstand der Aussenfläche in Abhängigkeit von einem Umlaufwinkel um die Längsachse (22)* nach aussen von der Längsachse (12) weg *vergrössert*, um den Verblockungsteil (10;10b;10c) des Verblockungselements (1;1b;1c) mit einer entsprechenden Innenkontur (200;200d;200e) der Aufnahme (2;2d;2e) verblocken zu können, **dadurch gekennzeichnet, dass** die umlaufende Aussenfläche wenigstens im Bereich der Klemmfläche (100;100b;100c) zumindest annähernd sphärisch, *paraboloidisch, ellipsoidisch oder hyperbolisch* ausgebildet ist und beim Verblocken eine radiale Verklemmung zwischen der Klemmfläche (100;100b;100c) und der Ausnehmung (20;20d;20e) der Aufnahme (2;2d;2e) bewirkt.

12. Verblockungselement nach Anspruch 11, bei welchem die umlaufende Aussenfläche drei gleichmässig entlang ihres Umfangs verteilte Klemmflächen (100;100b;100c) aufweist.

13. Verblockungselement nach einem der Ansprüche 11 oder 12, bei welchem die Kontur der Aussenfläche im Bereich der Klemmfläche (100;100b;100c) bzw. Klemmflächen in einer Azimutebene durch eine Funktion vom Typ r = a₂ + b₂√α beschrieben wird, wobei r den jeweiligen Abstand der Klemmfläche (100;100b;100c) von der Längsachse (12) bedeutet, a₂ und b₂ Konstanten sind, und α für den jeweiligen Azimutwinkel steht.

14. Verblockungselement nach einem der Ansprüche 11 oder 12, bei welchem die Kontur der Aussenfläche im Bereich der Klemmfläche (100;100b;100c) bzw. Klemmflächen in einer Azimutebene durch einen Teil einer logarithmischen Spirale, durch einen Teil einer Kreisbahn, durch einen Teil einer Evolvente, oder ähnlich, beschrieben wird.

15. Verblockungselement nach einem der Ansprüche 11 bis 14, bei welchem zumindest die Klemmfläche (100) mit einer Struktur (102) versehen ist.

16. Schraube (1;1b;1c), insbesondere Knochenschraube, mit einem Schraubenschaft (11), der wenigstens teilweise mit einem Gewinde (110) versehen ist, sowie mit einem über den Schaft (11) und das Gewinde (110) nach aussen vorstehenden Schraubenkopf (10;10b;10c), **dadurch gekennzeichnet, dass** die Schraube das Verblockungselement gemäss einem der Ansprüche 11 bis 15 bildet und der Schraubenkopf das Verblockungsteil des Verblockungselements bildet.

17. Schraube (1) nach Anspruch 16, bei welcher in den Klemmflächen (100) mindestens eine, vorzugsweise vier, rillenförmige Vertiefungen (103) vorgesehen sind, welche im wesentlichen in gleicher Richtung verlaufen und im wesentlichen die gleiche Steigung aufweisen wie das vom Schraubenschaft (11) nach aussen vorstehende Gewinde (110).

18. Knochenplatten-/Knochenschrauben- Sortiment, welches mindestens eine Knochenplatte (3) gemäss Anspruch 9 oder 10 sowie mindestens eine Knochenschraube (1;1b;1c) gemäss Anspruch 16 oder 17 umfasst.

19. Kombination eines *Aufnahmeelements* nach einem der Ansprüche 1 bis 8 mit einem Verblockungselement nach einem der Ansprüche 11 bis 15.

20. Kombination nach Anspruch 19, bei welcher die Aufnahme (2;2d;2e) *des Aufnahmeelements* wenigstens im Bereich der Ausnehmung (20;20d;20e) in Richtung der Längsachse (22) der Aufnahme betrachtet zylindrisch ausgebildet ist anstelle von annähernd sphärisch, *paraboloidisch, ellipsoidisch* oder *hyperboloidisch*.

21. Kombination nach Anspruch 19 oder 20, bei welcher das Verblockungselement eine Knochenplatte nach Anspruch 9 ist und das Verblockungselement eine Schraube nach einem der Ansprüche 16 oder 17 ist.

## Claims

1. A housing element having a housing (2; 2d; 2e) for a locking element (1; 1b; 1c), with a peripheral inner wall (200; 200d; 200e) which extends substantially in the direction of the longitudinal axis (22) of the housing and has at least one recess (20; 20d; 20e) in the region of which the distance of the inner wall from the longitudinal axis (22) increases outward as a function of the angle of revolution about the longitudinal axis (22), in order to be able to house and lock a locking part (10; 10b; 10c) of the locking element having a corresponding outer contour (100; 100b; 100c), **characterized in that** the peripheral inner wall is at least approximately spherical, paraboloid, ellipsoid or hyperboloid in the region of the recess (20; 20d; 20e) and, upon locking, permits a radial wedging between the recess (20; 20d; 20e) and the outer contour (100; 100b; 100c) of the locking part (10; 10b; 10c) of the locking element.

2. The housing element as claimed in claim 1, in which the recess (20; 20d) is arranged such that it extends in a direction perpendicular to the longitudinal axis (22) of the housing.

3. The housing element as claimed in claim 1, in which the recess (20e) is arranged such that it extends in the manner of a spatial spiral round the longitudinal axis (22) of the housing.

4. The housing element as claimed in one of claims 1 through 3, in which the peripheral inner wall has three recesses (20; 20d; 20e) which are distributed uniformly along its circumference.

5. The housing element as claimed in one of claims 1 through 4, in which the contour of the inner wall (200; 200d; 200e) in the region of the recess (20; 20d; 20e) or recesses is described by part of a function of the type r = a₁ + b₁ √α, where r is the respective distance of the inner wall (200; 200d; 200e) from the longitudinal axis (22) of the housing, a₁ and b₁ are constants, and α stands for the respective angle of revolution.

6. The housing element as claimed in one of claims 1 through 4, in which the contour of the inner wall (200; 200d; 200e) in the region of the recess (20; 20d; 20e) or recesses is described by part of a logarithmic spiral, by part of a circular trajectory, by part of an involute or the like.

7. The housing element as claimed in one of the preceding claims, in which the housing (2; 2d) has, on its peripheral inner wall, a runout contour (201; 201d) which adjoins the recess (20; 20d) or recesses in the circumferential direction and is used for guiding the locking element (1; 1b; 1c) out of the housing.

8. The housing element as claimed in one of the preceding claims, in which the housing (2; 2d; 2e) is provided with a countersink (202; 202d; 202e) for receiving a screw head (10a) with a spherical underside.

9. A bone plate (3) with plate apertures (30), at least one plate aperture (30) being provided with a housing (2; 2d; 2e) for a locking element (1; 1b; 1c) which is to be locked with the bone plate (3), for example for a bone screw (1; 1c), for a drill guide (1b) or for a fixing pin,
**characterized in that** the housing (2; 2d; 2e) is formed in a housing element according to one of the preceding claims.

10. The bone plate (3) as claimed in claim 9, in which at least one plate aperture is designed as an oblong hole in which the housing is formed in a housing element as claimed in one of claims 1 through 8.

11. A locking element (1; 1b; 1c) for introduction into a housing (2; 2d; 2e) of a housing element and for locking with this housing (2; 2d; 2e), in which locking element (1; 1b; 1c) a locking part (10; 10b; 10c), for example a head, is provided with a peripheral outer surface which extends substantially in the direction of a longitudinal axis (12) and has at least one clamping surface (100; 100b; 100c) in the region of which, viewed in an azimuth plane perpendicular to the longitudinal axis (12), the distance of the outer surface increases outward from the longitudinal axis (12) as a function of an angle of revolution about the longitudinal axis (22), in order to be able to lock the locking part (10; 10b; 10c) of the locking element (1; 1b; 1c) with a corresponding inner contour (200; 200d; 200e) of the housing (2; 2d; 2e), **characterized in that** the peripheral outer surface is at least approximately spherical, paraboloid, ellipsoid or hyperboloid, at least in the region of the clamping surface (100; 100b; 100c) and, upon locking, permits radial wedging between the clamping surface (100; 100b; 100c) and the recess (20; 20d; 20e) of the housing (2; 2d; 2e).

12. The locking element as claimed in claim 11, in which the peripheral outer surface has three clamping surfaces (100; 100b; 100c) which are distributed uniformly along its circumference.

13. The locking element as claimed in one of claims 11 and 12, in which the contour of the outer surface in the region of the clamping surface (100; 100b; 100c) or clamping surfaces is described in an azimuth plane by a function of the type r = a₂ + b₂ √α, where r is the respective distance of the clamping surface (100; 100b; 100c) from the longitudinal axis (12), a₂ and b₂ are constants, and α stands for the respective azimuth angle.

14. The locking element as claimed in one of claims 11 and 12, in which the contour of the outer surface in the region of the clamping surface (100; 100b; 100c) or clamping surfaces is described in an azimuth plane by part of a logarithmic spiral, by part of a circular trajectory, by part of an involute or the like.

15. The locking element as claimed in one of claims 11 through 14, in which at least the clamping surface (100) is provided with a structure (102).

16. A screw (1; 1b; 1c), in particular a bone screw, with a screw shank (11) which is provided at least partially with a thread (110), and with a screw head (10; 10b; 10c) which protrudes outward above the shank (11) and the thread (110), **characterized in that** the screw forms the locking element according to one of claims 11 through 15 and the screw head forms the locking part of the locking element.

17. The screw (1) as claimed in claim 16, in which at least one, but preferably four groove-like depressions (103) are provided in the clamping surfaces (100), which depressions (103) extend substantially in the same direction and have substantially the same pitch as the thread (110) protruding outward from the screw shank (11).

18. A set of bone plates and bone screws, containing at least one bone plate (3) as claimed in claim 9 or 10 and at least one bone screw (1; 1b; 1c) as claimed in claim 16 or 17.

19. A combination of a housing element as claimed in one of claims 1 through 8 to a locking element as claimed in one of claims 11 through 15.

20. The combination as claimed in claim 19, in which the housing (2; 2d; 2e) of the housing element, viewed in the direction of the longitudinal axis (22) of the housing, is cylindrical, at least in the region of the recess (20; 20d; 20e), instead of being approximately spherical, paraboloid, ellipsoid or hyperboloid.

21. The combination as claimed in claim 19 or 20, in which the locking element is a bone plate as claimed in claim 9 and the locking element is a screw as claimed in one of claims 16 and 17.

## Revendications

1. Elément de réception qui présente un logement (2 ; 2d ; 2e) pour un élément de blocage (1 ; 1b ; 1c) et une paroi intérieure périphérique (200 ; 200d ; 200e) qui s'étend essentiellement dans la direction de l'axe longitudinal (22) du logement et qui présente au moins une découpe (20 ; 20d ; 20e) dans la zone de laquelle la distance entre la paroi intérieure et l'axe longitudinal (22) s'agrandit lorsque l'on s'éloigne vers l'extérieur en fonction de l'angle périphérique autour de l'axe longitudinal (22), pour loger et pouvoir bloquer une pièce de blocage (10 ; 10b ; 10c) de l'élément de blocage à contour extérieur (100 ; 100b ; 100c) correspondant,
**caractérisé en ce que**
dans la zone occupée par le logement (20 ; 20d ; 20e), la paroi périphérique intérieure a la configuration au moins approximative d'une sphère, d'un paraboloïde, d'un ellipsoïde ou d'un hyperboloïde et
**en ce que** lors du blocage, elle a pour effet un serrage radial entre la découpe (20 ; 20d ; 20e) et le contour extérieur (100 ; 100b ; 100c) de la pièce de blocage (10 ; 10b ; 10c) de l'élément de blocage.

2. Elément de réception selon la revendication 1, dans lequel la découpe (20 ; 20d) est disposée de telle sorte qu'elle se déploie dans une direction perpendiculaire à l'axe longitudinal (22) du logement.

3. Elément de réception selon la revendication 1, dans lequel la découpe (20e) est disposée de telle sorte qu'elle entoure en spirale tridimensionnelle l'axe longitudinal (22) du logement.

4. Elément de réception selon l'une des revendications 1 à 3, dans lequel trois découpes (20 ; 20d ; 20e) sont réparties uniformément à la périphérie de la paroi intérieure périphérique.

5. Elément de réception selon l'une des revendications 1 à 4, dans lequel le contour de la paroi intérieure (200 ; 200d ; 200e) est décrit dans la partie occupée par la découpe (20 ; 20d ; 20e) ou les découpes par une partie d'une fonction de type r = a₁ + b₁√α dans laquelle r représente la distance entre la paroi intérieure (200 ; 200d ; 200e) et l'axe longitudinal (22) du logement, a₁ et b₁ sont des constantes et α représente l'angle périphérique.

6. Elément de réception selon l'une des revendications 1 à 4, dans lequel le contour de la paroi intérieure (200 ; 200d ; 200e) est décrit dans la partie occupée par la découpe (20 ; 20d ; 20e) ou les découpes par une partie d'une spirale logarithmique, une partie d'une piste circulaire, une partie d'une développante ou similaires.

7. Elément de réception selon l'une des revendications précédentes, dans lequel le logement (2 ; 2d) présente sur sa paroi périphérique intérieure un contour de sortie (201 ; 201d) qui se raccorde à la découpe (20 ; 20d) ou aux découpes pour guider l'élément de blocage (1 ; 1b ; 1c) hors du logement.

8. Elément de réception selon l'une des revendications précédentes, dans lequel le logement (2 ; 2d ; 2e) est doté d'un creux (202 ; 202d ; 202e) pour la réception d'une tête de vis (10a), le côté inférieur de la tête étant sphérique.

9. Plaque d'ostéosynthèse (3) présentant des trous (30) de plaque, au moins un trou (30) de plaque étant doté d'un logement (2 ; 2d ; 2e) pour un élément de blocage (1 ; 1b ; 1c) qui doit être bloqué sur la plaque d'ostéosynthèse (3), par exemple pour une vis d'ostéosynthèse (1 ; 1c), pour un guide d'alésage (1b) ou une tige de fixation,
**caractérisée en ce que**
le logement (2 ; 2d ; 2e) est configuré en élément de réception selon l'une des revendications précédentes.

10. Plaque d'ostéosynthèse (3) selon la revendication 9, dans laquelle au moins un trou de plaque est configuré comme trou oblong dans lequel le logement est configuré en élément de réception selon l'une des revendications 1 à 8.

11. Elément de blocage (1 ; 1b ; 1c) destiné à être inséré dans un logement (2 ; 2d ; 2e) d'un élément de réception et à être bloqué dans ce logement (2 ; 2d ; 2e), l'élément de blocage (1 ; 1b ; 1c) étant une pièce de blocage (10 ; 10b ; 10c), par exemple une tête, dont la surface extérieure périphérique s'étend essentiellement dans la direction d'un axe longitudinal (12) et présente au moins une surface de serrage (100 ; 100b ; 100c), 1a distance, vue dans un plan azimutal perpendiculaire à l'axe longitudinal (12), entre la surface extérieure et l'axe longitudinal (12) s'agrandissant lorsque l'on s'en éloigne vers l'extérieur en fonction de l'angle de rotation autour de l'axe longitudinal (22), pour pouvoir bloquer la pièce de blocage (10 ; 10b ; 10c) de l'élément de blocage (1 ; 1b ; 1c) sur un contour intérieur (200 ; 200d ; 200e) correspondant du logement (2 ; 2d ; 2e),
**caractérisé en ce que**
au moins dans la zone occupée par la surface de serrage (100 ; 100b ; 100c), la surface périphérique extérieure a la configuration au moins approximative d'une sphère, d'un paraboloïde, d'un ellipsoïde ou d'un hyperboloïde et
**en ce que** lors du blocage, elle a pour effet un serrage radial entre la surface de serrage (100 ; 100b ; 100c) et la découpe (20 ; 20d ; 20e) du logement (2 ; 2d ; 2e).

12. Elément de blocage selon la revendication 11, dans lequel trois surfaces de serrage (100 ; 100b ; 100c) sont réparties uniformément à la périphérie de la surface extérieure périphérique.

13. Elément de blocage selon l'une des revendications 11 ou 12, dans lequel, dans la zone occupée par la surface de serrage (100 ; 100b ; 100c) ou des surfaces de serrage, le contour de la surface extérieure dans un plan azimutal est décrit par une fonction du type r = a₂ + b₂√α, dans laquelle r représente la distance entre la surface de serrage (100 ; 100b ; 100c) et l'axe longitudinal (12), a₂ et b₂ sont des constantes et α représente l'angle azimutal.

14. Elément de blocage selon l'une des revendications 11 ou 12, dans lequel, dans la zone occupée par la surface de serrage (100 ; 100b ; 100c) ou les surfaces de serrage, le contour de la surface extérieure dans un plan azimutal est décrit par une partie d'une spirale logarithmique, une partie d'une piste circulaire, une partie d'une développante ou similaires.

15. Elément de blocage selon l'une des revendications 11 à 14, dans lequel au moins la surface de serrage (100) est dotée d'une structuration (102).

16. Vis (1 ; 1b ; 1c), en particulier vis d'ostéosynthèse, dotée d'une tige de vis (11) dont au moins une partie présente un filet (110) et d'une tête de vis (10 ; 10b ; 10c) qui déborde vers l'extérieur au-delà de la tige (11) et du filet (110),
**caractérisée en ce que**
la vis forme l'élément de blocage selon l'une des revendications 11 à 15 et la tête de la vis la pièce de blocage de l'élément de blocage.

17. Vis (1) selon la revendication 16, dans laquelle au moins un et de préférence quatre creux (103) en forme de rainure qui s'étendent essentiellement dans la même direction et qui présentent essentiellement le même pas que le filet (110) qui déborde à l'extérieur de la tige (11) de la vis sont prévus dans les surfaces de serrage (100).

18. Assortiment de plaques d'ostéosynthèse et de vis d'ostéosynthèse qui comporte au moins une plaque d'ostéosynthèse (3) selon les revendications 9 ou 10 ainsi qu'au moins une vis d'ostéosynthèse (1 ; 1b ; 1c) selon les revendications 16 ou 17.

19. Combinaison d'un élément de réception selon l'une des revendications 1 à 8 avec un élément de blocage selon l'une des revendications 11 à 15.

20. Combinaison selon la revendication 19, dans laquelle au moins dans la zone occupée par la découpe (20 ; 20d ; 20e), le logement (2 ; 2d ; 2e) de l'élément de réception a la configuration d'un cylindre plutôt que sensiblement d'une sphère, d'un paraboloïde, d'un ellipsoïde ou d'un hyperboloïde dans la direction de l'axe longitudinal (22) du logement.

21. Combinaison selon les revendications 19 ou 20, dans laquelle l'élément de blocage est une plaque d'ostéosynthèse selon la revendication 9 et l'élément de blocage une vis selon l'une des revendications 16 ou 17.
